(19) Europäisches Patentamt
European Patent Office
Office européen des brevets

(11) **EP 3 160 633 B1**

(12) **EUROPÄISCHE PATENTSCHRIFT**

(45) Veröffentlichungstag und Bekanntmachung des Hinweises auf die Patenterteilung:
**06.06.2018 Patentblatt 2018/23**

(21) Anmeldenummer: **15730516.0**

(22) Anmeldetag: **23.06.2015**

(51) Int Cl.:
**B01J 8/08** (2006.01)     **B01J 8/18** (2006.01)
**B01J 8/42** (2006.01)     **B01J 8/00** (2006.01)
**B01J 8/02** (2006.01)     **B01J 19/08** (2006.01)

(86) Internationale Anmeldenummer:
**PCT/EP2015/064094**

(87) Internationale Veröffentlichungsnummer:
**WO 2015/197608 (30.12.2015 Gazette 2015/52)**

(54) **VERFAHREN ZUR SELBSTREGULATION EINES SYSTEMS**

METHOD FOR SELF-REGULATION OF A SYSTEM

PROCÉDÉ POUR L'AUTO-RÉGULATION D'UN SYSTÈME

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.06.2014 EP 14173612**

(43) Veröffentlichungstag der Anmeldung:
**03.05.2017 Patentblatt 2017/18**

(73) Patentinhaber: **BASF SE**
**67056 Ludwigshafen am Rhein (DE)**

(72) Erfinder:
• **GARELLA, Dominik**
  **69168 Wiesloch (DE)**
• **SILVA, Viviana**
  **67117 Limburgerhof (DE)**
• **LANG, Tobias**
  **69221 Dossenheim (DE)**
• **GARCIA PALACIOS, Javier**
  **50667 Köln (DE)**

(56) Entgegenhaltungen:
DE-A1-102009 028 856     FR-A1- 2 691 718
US-A1- 2003 086 839

## Beschreibung

Die Erfindung betrifft ein Verfahren zur Selbstregulation eines Systems, wobei ein Magnetfeld eingesetzt wird und die magnetischen Eigenschaften magnetisierbarer und/oder magnetischer Partikel geändert werden.

Magnetfeldunterstützte Verfahren nutzen ein Magnetfeld um einzelne Schritte des Verfahrens zu verbessern oder überhaupt umzusetzen. Beispiele für magnetisch unterstützte Prozesse sind magnetisch stimulierte Bioprozesse, magnetische Wechselfelder zum Beheizen, die Aktivierung von Radikalreaktionen und auch Magnetrührer.

Magnetfeldunterstützte Reaktoren (magnetically enhanced reactors, MER), auch magnetisch unterstütze Reaktoren genannt, insbesondere magnetfeldunterstützte Wirbelschichtsysteme, sind in der Literatur vielfach als attraktive Alternativen oder Ergänzungen zu Festbettreaktoren, Wirbelschichtreaktoren und Suspensionsreaktoren, auch Slurryreaktoren genannt, beschrieben. Hierbei wirkt ein externes Magnetfeld auf magnetisierbare und/oder magnetische Partikel, die in einer Wirbelschicht enthalten sind. Magnetische Eigenschaften der magnetisierbaren und/oder magnetischen Partikel sind unter anderem von der chemischen Zusammensetzung der magnetisierbaren und/oder magnetischen Partikel und/oder ihrer Temperatur abhängig. Die Wirbelschichtsysteme können aus Gas-Feststoff-Gemischen oder Gas-Flüssigkeit-Gemischen bestehen. Ebenso ist die gleichzeitige Anwesenheit von Gasen, Flüssigkeiten und Feststoffen in einer Wirbelschicht denkbar.

Häufig werden in MERs magnetisierbare und/oder magnetische Partikel mit ferromagnetischen Eigenschaften als Katalysatoren oder als Trägermaterial für katalytisch aktive Substanzen eingesetzt, die in einem kontinuierlich betriebenen Reaktor fluidisiert oder verwirbelt werden. Ein den Reaktor durchströmendes Fluid übt auf die magnetisierbaren und/oder magnetischen Partikel im MER eine Kraft in Strömungsrichtung des Fluids aus, wie es zum Beispiel auch für Wirbelschichtsysteme der Fall ist. Andererseits wirken Magnetkräfte eines angelegten Magnetfeldes und im Normalfall auch die Schwerkraft dieser durch das strömende Fluid bedingten Bewegung der magnetisierbaren und/oder magnetischen Partikel entgegen.

Die Vorteile von magnetfeldunterstützten Wirbelschichten sind eine intensivierte Prozesskontrolle sowie im Vergleich zu Festbettreaktoren ein niedriger Druckverlust und eine bessere Stoffübertragung und Wärmeübertragung und im Vergleich zu nicht magnetfeld unterstützten Wirbelschichten eine geringere Rückvermischung, ein höher Durchsatz, die Vermeidung von Blasenbildung und By-Pässen des Fluids, ein möglicher Betrieb mit kleineren Partikelgrößen bis in den Nanobereich und ein geringerer Austrag und Abrieb der in der Wirbelschicht enthaltenen Partikel. Mit einer magnetfeld unterstützten Wirbelschicht können Vorteile eines Festbettes, wie ein guter Stoffübergang zwischen fluider Phase und Feststoffphase und eine enge Verweilzeitverteilung der Phasen, und Vorteile einer Wirbelschicht, wie hohe Wärmeübergangskoeffizienten und Stoffübergangskoeffizienten, eine homogene Temperaturverteilung und die Möglichkeit zur kontinuierlichen Zufuhr und Abfuhr von Feststoff, vereint werden. Durch die magnetische Unterstützung kann die Feststoffbewegung besser kontrolliert werden. Eine Einwirkung des Magnetfeldes auf die magnetischen Partikel bietet somit einen zusätzlichen Freiheitsgrad um den Fluidisationszustand der magnetisierbaren und/oder magnetischen Partikel zu beeinflussen. Darüber hinaus bietet der Einsatz eines Magnetfeldes Alternativen zur pneumatischen Förderung.

Das Zusammenspiel von veränderlichen magnetischen Eigenschaften der magnetisierbaren und/oder magnetischen Partikel und einer Bewegung der magnetisierbaren und/oder magnetischen Partikel, die wiederum von den magnetischen Eigenschaften abhängig ist, kann zur Temperaturführung und Reaktionsführung im Reaktor genutzt werden.

Die DE 10 2007 059 967 A1 offenbart ein Verfahren zur Durchführung chemischer Reaktionen mit Hilfe eines induktiv erwärmten Heizmediums. Dazu wird das Reaktionsmedium in Kontakt mit einem durch elektromagnetische Induktion erwärmbaren festen Heizmedium gebracht. Das Heizmedium kann ferromagnetisch sein und eine Curie-Temperatur aufweisen, welche nicht mehr als 20°C von der Reaktionstemperatur abweicht. Damit ist eine Selbstregelung der Temperatur des Heizmediums gegeben und eine Überhitzung durch das Heizmedium ausgeschlossen, da das Heizmedium nicht induktiv auf eine Temperatur erwärmt werden kann, welche höher ist als die Curie-Temperatur des Heizmediums.

Die DE 696 03 270 T2 hat ein magnetisches Trennverfahren für Eisencarbid zum Gegenstand. Das aus einem Reaktionsraum entnommene Gemisch aus Eisencarbid, Eisen, Eisenoxiden und Begleitmineralien wird unter Anwendung unterschiedlicher Temperaturen und nacheinander eingesetzten magnetischen Feldern aufgetrennt, wobei die verschiedenen Curie-Temperaturen der einzelnen Bestandteile des Gemisches ausgenutzt werden.

Ebenso ist in der FR 2691718 A1 die Trennung von eisenhaltigem und nicht eisenhaltigem Material mit einem magnetischen Feld nach Abkühlung auf eine Temperatur unterhalb der Curie-Temperatur des eisenhaltigen Materials beschrieben.

Aus der WO 2006/071527 ist ein Reaktor zur Synthese von Nanostrukturen bekannt, worin ferromagnetische Partikel eingesetzt werden, die eine der Reaktionstemperatur im Wesentlichen gleiche Curie-Temperatur aufweisen und die einen Katalysator enthalten. Die Partikel werden induktiv erwärmt.

Die DE 3642557 A1 beschreibt eine Wirbelschicht für die Pyrolyse von festem kohlenstoffhaltigem Material, wobei als Wärmeübertragungsmedium magnetisierbare und/oder magnetische Partikel eingesetzt werden. Die magnetisierbaren und/oder magnetischen Partikel werden separat erhitzt und dann der Pyrolysestufe zugeführt. Nach der Pyrolyse werden die verbleibenden Feststoffe falls nötig auf eine Temperatur abgekühlt, welche unterhalb der Curie-Temperatur der magnetisierbaren und/oder magnetischen Partikel liegt. Anschließend werden die magnetisierbaren und/oder magnetischen Partikel von den Pyrolyseprodukten durch Einsatz eines magnetischen Feldes getrennt und die magnetisierbaren und/oder magnetischen Partikel werden in die Pyrolysestufe zurückgeführt.

Die US 4354856 hat ein Verfahren zur Abtrennung von magnetischen Partikeln aus einem Fluid zum Gegenstand. Um die magnetischen Partikel anzuziehen wird ein ferromagnetisches Element eingesetzten, welches anschließend auf eine Temperatur gleich oder höher als die Curie-Temperatur des ferromagnetischen Elementes erwärmt wird um die magnetischen Partikel wieder von dem ferromagnetischen Element zu lösen.

Die FR 2 676 374 A1 offenbart ein kontinuierlich betriebenes magnetfeldunterstütztes Partikelbett, wobei die Partikel des Bettes im laufenden Verfahren ausgetauscht werden, indem diese aus dem Partikelbett abgesaugt und frische Partikel dem Partikelbett zugeführt werden.

Aus der EP 0 115 684 A1 ist ein Verfahren zur Durchführung von Reaktionen in einem magnetfeldunterstütztem Partikelbett mit kontrolliertem Reaktionsgeschwindigkeitsprofil bekannt. Die Partikel sind magnetisierbar, können katalytische Eigenschaften aufweisen und dienen der Wärmzufuhr oder Wärmeabfuhr. Zur Erhöhung des Umsatzes, der Reaktivität und/oder der Produktausbeute wird das Partikelbett in Form einer Pfropfenströmung durch den Reaktor gefördert, nach Austritt aus dem Reaktor gegebenenfalls regeneriert und in den Reaktor zurückgeführt.

Die EP 0 021 854 A1, US 4294688 und US 4292171 haben jeweils Verfahren zum Gegenstand, in denen magnetisierbare, teils katalytisch aktive Partikel einer magnetfeldunterstützten Wirbelschicht kontinuierlich entzogen sowie regeneriert und zurückgeführt werden.

Die DE 29 50 621 A1 beschreibt eine Katalysatorzusammensetzung aus passivierten magnetischen Legierungen für den Einsatz in magnetisch stabilisierten Wirbelschichten für Hochtemperatur-Anwendungen. Die Katalysatorzusammensetzung enthält Aluminium, Silizium und/oder Chrom, besitzt eine hohe Curie-Temperatur und ist in einer korrodierenden Umgebung stabil.

Die in den Dokumenten des Standes der Technik offenbarten magnetfeldunterstützten Wirbelschichten und Magnetfeld basierten Trennverfahren ziehen keinen Nutzen aus der Abhängigkeit der magnetischen Eigenschaften eines Feststoffs von der Temperatur oder Zusammensetzung zur Selbstregulation eines Systems, wobei der Transport von magnetisierbaren und/oder magnetischen Partikeln zur Temperaturführung oder Reaktionsführung eingesetzt wird. Zum Beispiel bedarf es in dem in der EP 0 115 684 A1 beschriebenen Verfahren zur Temperaturführung eines hohen prozesstechnischen Aufwandes, der die Einstellung eines gewünschten Temperaturniveaus über die Bestimmung von Förderraten von außen steuert. Die Änderung der magnetischen Eigenschaften wird zum Beispiel in der US 4354856, DE 696 03 270 T2 oder DE 3642557 A1 durch Änderungen außerhalb des Systems herbeigeführt um ein Trennprinzip überhaupt realisieren zu können. In der DE 10 2007 059 967 wird zwar eine Überhitzung durch induktiven Wärmeeintrag verhindert, jedoch kann hier kein selbstregelnder Einfluss auf die Zusammensetzung der Mischung im Reaktionsraum genommen werden, der eine Überhitzung auch aus anderen Gründen als dem induktiven Wärmeeintrag verhindern könnte.

Aufgabe der vorliegenden Erfindung ist es, ein Verfahren zur Selbstregulation eines Systems bereitzustellen, wobei die Zusammensetzung eines Gemisches in einem Bilanzraum entsprechend aktuellen Temperaturbedingungen und/oder einem aktuellen Reaktionsfortschritt angepasst wird ohne auf eine externe Regelung, also eine Peripherie außerhalb des Bilanzraumes, angewiesen zu sein.

Gelöst wird die Aufgabe durch ein Verfahren zur Selbstregulation eines Systems, umfassend folgende Schritte:

(I) Nutzung eines Magnetfelds um magnetisierbare und/oder magnetische Partikel aus einem Bilanzraum zu transportieren oder in dem Bilanzraum zu lokalisieren,

(II) Änderung von magnetischen Eigenschaften der magnetisierbaren und/oder magnetischen Partikel, die ferromagnetisch oder paramagnetisch sind, in dem Bilanzraum durch Änderung einer Temperatur Tp der magnetisierbaren und/oder magnetischen Partikel oder durch Änderung der Zusammensetzung der magnetisierbaren und/oder magnetischen Partikel.

Das Magnetfeld ist bevorzugt ein externes Magnetfeld, welches durch unterschiedliche, dem Fachmann bekannte Anordnungen von Spulen, insbesondere elektrischen Spulen, erzeugt wird. Extern bedeutet in diesem Zusammenhang, dass das Magnetfeld nicht in dem Bilanzraum erzeugt wird, aber das Magnetfeld in dem Bilanzraum existiert. Je nach Anordnung und Ausgestaltung der Spulen weist das Magnetfeld unterschiedliche Orientierungen, Feldstärken und Homogenitäten auf. Eine magnetische Feldstärke H kann durch folgende Gleichung beschrieben werden:

$$H = I \cdot \frac{n}{L}, \qquad (1)$$

wobei I die Stromstärke, n die Anzahl der Wicklungen und L die Länge der Spule ist. Die Einheit der magnetischen Feldstärke H ist A/m. Die magnetische Feldstärke steht in linearem Zusammenhang mit der magnetischen Induktion B:

$$B = \mu_0 \cdot \mu_r \cdot H, \qquad (2)$$

wobei $\mu_0$ die magnetische Feldkonstante, also die magnetische Permeabilität des Vakuums, ist und $\mu_r$ die Permeabilitätszahl, welche ein Maß für den Einfluss des im Magneten enthaltenen Materials auf das Magnetfeld darstellt.

Das Magnetfeld übt eine Magnetkraft $F_m$ auf magnetische oder magnetisierbare Partikel aus, die von den Partikeleigenschaften und den Eigenschaften des externen Feldes abhängig ist:

$$F_m = \mu_0 \cdot V_p \cdot M_p \cdot \nabla H, \qquad (3)$$

wobei $\mu_0$ die magnetische Feldkonstante, $V_p$ das Partikelvolumen, $M_p$ die Magnetisierung des Partikels und $\nabla H$ der Gradient der magnetischen Feldstärke am Ort des Partikels ist. Die Magnetisierung des Partikels wiederum ist von der magnetischen Feldstärke H des externen magnetischen Feldes abhängig:

$$M_p = \kappa \cdot H \qquad (4).$$

Die magnetische Suszeptibilität $\kappa$ ist ein Maß für die Magnetisierbarkeit von Materie in einem externen magnetischen Feld. Die magnetische Suszeptibilität $\kappa$ hängt im Fall von ferri- und ferromagnetischem Material unter anderem von der Partikelgröße, der Partikelform und der Feldintensität ab. Für diamagnetisches und paramagnetisches Material ist die magnetische Suszeptibilität $\kappa$ konstant.

Aus Kombination der Gleichungen (3) und (4) ergibt sich, dass die von dem Magnetfeld auf magnetische oder magnetisierbare Partikel, die spezifische Eigenschaften besitzen, ausgeübte Magnetkraft $F_m$ von der magnetischen Feldstärke H und dem Gradienten $\nabla H$ der magnetischen Feldstärke am Ort des jeweiligen Partikels abhängig ist:

$$F_m \propto H \cdot \nabla H \qquad (5).$$

Das Verhalten einer magnetisch unterstützen Wirbelschicht wird durch Schwerkraft $F_{SK}$, Magnetkraft $F_m$, Strömungswiderstand Fw und Auftrieb $F_A$ bestimmt. Die Kräftebilanz unter Vernachlässigung von Stößen der Partikel untereinander für ein einzelnes Partikel, welches sich in einer magnetisch unterstützen Wirbelschicht mit externem Magnetfeld befindet, ergibt sich zu:

$$F_W + F_A = F_{SK} + F_m \qquad (6).$$

Der tatsächliche Einfluss des Magnetfeldes auf die Partikel ist von der magnetischen Feldstärke H und der Homogenität des Magnetfeldes abhängig. Unabhängig von der Homogenität des Feldes induziert das externe Magnetfeld Anziehungskräfte zwischen den einzelnen Partikeln, die zu Aggregation und Kettenbildung führen. Das erzeugte Magnetfeld in einem Volumen, das von der Wirbelschicht eingenommen wird, kann homogen oder inhomogen sein. Ein homogenes Magnetfeld weist zueinander parallele Feldlinien in der Wirbelschicht oder im Reaktor auf. In diesem Fall nimmt das Maß der Fluidisierung der Wirbelschicht durch das Magnetfeld ab. Die durch das Magnetfeld induzierten Kräfte führen zu Partikelagglomeration, so dass sich größere Aggregate bilden, die sich wie schwerere Partikel in der Wirbelschicht verhalten. Dies wird in einer magnetfeldunterstützten Wirbelschicht ausgenutzt. In einem inhomogenen Magnetfeld, das durch eine Reihe von verschiedenen Spulen erzeugt wird, sind die Magnetfeldlinien gebogen und eine auf eine Querschnittsfläche des Reaktors bezogene Feldliniendichte ist über einer Reaktorhöhe nicht konstant. Eine der Schwerkraft ähnliche Kraft kann im Reaktor erzeugt werden, wenn aus dem Magnetfeld resultierende Kraftvektoren einer aufsteigenden Fluidströmung entgegengerichtet sind.

Durch Variation der magnetischen Feldstärke können unterschiedliche Strömungszustände in einem Reaktor erzeugt werden. Bei großer magnetischer Feldstärke nähert sich der Strömungszustand dem Strömungszustand eines Festbettes an, bei kleiner magnetischer Feldstärke ist das Bett vollständig fluidisiert. Eine magnetisch unterstütze Wirbelschicht kann auch als eine partiell fixierte Wirbelschicht in einem strömenden Fluid beschrieben werden.

Im Allgemeinen werden magnetisch unterstütze Reaktoren mit konstanten Magnetfeldern betrieben. Ebenso ist der Einsatz alternierender Felder möglich. Mit alternierenden Feldern kann eine Wirbelschicht erzeugt werden, auch wenn die Strömungsgeschwindigkeit des Fluids allein noch nicht zu einer Fluidisierung führen würde.

In einer weiteren Ausführungsform können in dem Bilanzraum oder an den Grenzen des Bilanzraumes Siebe und/oder Zwischenböden eingesetzt werden, deren Lochdurchmesser größer sind als die Durchmesser der magnetisierbaren und/oder magnetischen Partikel. Durch Anlegen des Magnetfelds kann es zu Bildung von Aggregaten aus den magnetisierbaren und/oder magnetischen Partikeln kommen, die wiederum einen größeren Durchmesser als die Löcher der Siebe besitzen. Auf diese Weise kann durch das Magnetfeld die Passage der magnetisierbaren und/oder magnetischen Partikel durch die Siebe und/oder Zwischenböden gesteuert werden.

Bezüglich der Richtung der magnetischen Feldlinien in einer Wirbelschicht werden axiale Felder, deren Feldlinien parallel zur Flussrichtung des Fluids verlaufen, und transversale Felder, welche senkrecht zur Flussrichtung des Fluids verlaufen, unterschieden. Bevorzugt werden transversale Magnetfelder eingesetzt, welche durch Sattelspulen oder Helmholtzspulen erzeugt werden können, da hochexpandierte Festbetten erreicht werden können und eine Kanalbildung in der Wirbelschicht im Vergleich zum Einsatz von axialen Feldern weniger leicht auftritt. Darüber hinaus weisen transversale Felder den Vorteil auf, dass in ca. 99% des Volumens, in dem das Magnetfeld herrscht, ein homogenes Magnetfeld vorliegen kann. Der Volumenanteil eines homogenen Magnetfeldes beträgt für axiale Felder üblicherweise nur ca. 30%. Bei axialen Feldern ist das Magnetfeld in seinem Zentrum homogen und die Magnetfeldstärke nimmt in Richtung der Wicklungen der Spule zu. Wenn ein homogenes Magnetfeld über den gesamten Querschnitt eines Reaktors gewünscht ist, so muss im Fall von axialen Feldern der Spulendurchmesser deutlich größer als der Reaktordurchmesser sein. Daraus ergeben sich sowohl erhöhte Materialkosten sowie ein großes Arbeitsvolumen und ein erhöhter Energiebedarf.

Magnetisch stabilisierte Wirbelschichten erfordern häufig ein homogenes Magnetfeld. In magnetisch stabilisierten Wirbelschichten bilden sich auf Grund von interpartikularen Magnetkräften Aggregate, die sich wie schwerere Partikel in der Wirbelschicht verhalten und zu einer stabileren Fluidisierung führen. In einer magnetfeldunterstützten Wirbelschicht hingegen treten lediglich schwache Kräfte zwischen den einzelnen Partikeln auf.

Die magnetfeldunterstützte Wirbelschicht enthält magnetische oder magnetisierbare Partikel, so dass eine Wechselwirkung zwischen dem Magnetfeld und den Partikeln möglich ist. Die magnetisch unterstütze Wirbelschicht kann ausschließlich magnetische und/oder magnetisierbare Partikel oder eine Mischung aus magnetisierbaren und/oder magnetischen Partikel und nicht-magnetischem beziehungsweise aus nicht magnetisierbarem Material enthalten. Die Mischung enthält bevorzugt mindestens 10 Vol.-%, insbesondere zwischen 10 Vol.-% und 30 Vol.-% bezüglich des Bettvolumens magnetische und/oder magnetisierbare Partikel. Die magnetisierbaren und/oder magnetischen Partikel weisen bevorzugt variable magnetische Eigenschaften auf, die paramagnetisch oder ferromagnetisch sein können. Paramagnetische Materialien besitzen eine Permeabilität, die größer als 1 ist. In paramagnetischen Materialien sind magnetische Momente regellos angeordnet und ordnen sich teilweise in einem externen Magnetfeld solange das externe Magnetfeld existiert. Paramagnetische Materialien werden in ein inhomogenes magnetisches Feld gezogen. Sie haben die Tendenz zur Wanderung in ein stärkeres Feld. Im Vergleich zu paramagnetischen Materialien kann eine Magnetisierung von ferromagnetischen Materialien als stabil bezeichnet werden. Ferromagnetische Materialien richten ihre Elementarmagnete parallel zueinander aus. Sie können von einem Magnetpol eines äußeren Magnetfelds angezogen werden. Ferromagnetische Materialien zeigen in einem externen Magnetfeld eine eigene Magnetisierung. Beispiele für bei Raumtemperatur ferromagnetische Elemente sind Eisen, Nickel und Cobalt. Die magnetischen Eigenschaften sind abhängig von der Temperatur $T_p$ der magnetisierbaren und/oder magnetischen Partikel und/oder der Zusammensetzung der magnetisierbaren und/oder magnetischen Partikel. Je nach der Temperatur $T_p$ der magnetisierbaren und/oder magnetischen Partikel stehen die magnetisierbaren und/oder magnetischen Partikel unter dem Einfluss des Magnetfeldes oder nicht.

Unter einer Curie-Temperatur $T_c$ wird die Temperatur der magnetisierbaren und/oder magnetischen Partikel verstanden, bei deren Überschreitung ferromagnetisches Material paramagnetisch wird und damit seine magnetischen Eigenschaften verliert. In einer magnetfeldunterstützten Wirbelschicht können die magnetisierbaren und/oder magnetischen Partikel auch bereits dem Einfluss des Magnetfelds entzogen werden, wenn die Temperatur $T_p$ der magnetisierbaren und/oder magnetischen Partikel die Curie-Temperatur $T_c$ noch nicht erreicht hat, wenn bei dieser Temperatur $T_p$ die Magnetkraft der Partikel so gering ist, dass die hydrodynamischen Kräfte im System überwiegen und für die Bewegung der magnetisierbaren und/oder magnetischen Partikel vorherrschend sind.

Die magnetisierbaren und/oder magnetischen Partikel können auf unterschiedliche Weise aufgebaut sein. Sie können beispielsweise aus Verbundmaterial bestehen oder nach dem Kern-Schale-Prinzip aufgebaut sein, wobei ein magnetisierbarer Kern mit einem nicht-magnetischen Material beschichtet ist, beispielsweise beschrieben in Dong et al., Selective acetylene hydrogenation over core-shell magnetic Pd-supported catalysts in a magnetically stabilized bed, American Institute of Chemical Engineers Journal, 2008, Volume 54, Nummer 5, Seiten 1358 bis 1364 und Fu et al., Preparation and properties of magnetic alumina microspheres with a γ-Fe2O3/SiO2 core and Al2O3 shell, Journal of Natural Gas Chemistry, 2011, Volume 20, Nummer 1, Seiten 72 bis 76. Das nicht-magnetische Material kann eine katalytisch aktive Substanz enthalten oder eine katalytisch aktive Substanz kann auf der nicht-magnetischen Beschichtung immobilisiert werden.

Cu-Ni-Ferrite sind bevorzugte Materialen für die magnetisierbaren und/oder magnetischen Partikel, da sie die Einstellung gewünschter Curie-Temperaturen Tc über die Wahl des Verhältnisses von Kupfer zu Nickel erlauben und darüber hinaus katalytisch aktive Materialen für Hydrierreaktionen wie die Hydrierung von Nitrobenzol sind, beispielsweise beschreiben in der WO 2008/034770 A1.

Typische Curie-Temperaturen Tc von eingesetzten magnetisierbaren und/oder magnetischen Partikeln liegen zwischen 300°C und 900°C. Die Curie-Temperatur Tc von Mischmaterialien ist von dem Herstellungsverfahren der Mischmaterialien abhängig. Ein bevorzugtes Mischmaterial für magnetisierbare und/oder magnetische Partikel enthält Eisen, Kupfer und Nickel. Bevorzugt beträgt das molare Verhältnis von Nickel zu Kupfer zwischen 4 und 0,25. Ein bevorzugtes Herstellungsverfahren für die magnetisierbaren und/oder magnetischen Partikel umfasst ein Mischen von Ausgangmaterialien und eine Kalzination bei mindestens 900°C über einen Zeitraum von mehreren Stunden. Bevorzugt enthalten die magnetisierbaren und/oder magnetischen Partikel keine kristalline Magnetit-Phase. Dies ist vorteilhaft, da die Curie-Temperatur Tc von Magnetit mit Werten zwischen 560°C und 590°C vergleichsweise hoch ist und damit höher ist als zum Beispiel ein angestrebter Bereich einer Reaktionstemperatur für die Hydrierung von Nitrobenzol von 250°C bis 350°C.

Tabelle 1 Beispiele für Materialien mit unterschiedlichen Curie-Temperaturen

| Material | Curie-Temperatur [°C] |
|---|---|
| Co | 1121 |
| Fe | 770 |
| Ni | 358 |
| Magnetit (Fe$_3$O$_4$) | 578 |
| γ-Fe$_3$O$_4$ (Maghemit) | 858 |
| Fe-Ni (46 Gew.-% Ni) | 733 |
| Fe-Co (50 Gew.-% Co) | 998 |
| Li-Ferrit | 631 |
| Ni-Ferrit | 575 bis 597 |
| Co-Ferrit | 495 bis 520 |
| Cu-Ferrit | 410 bis 490 |
| Mn-Ferrit | 295 bis 303 |
| Ni$_{0.8}$Cu$_{0.2}$Fe$_2$O$_4$ | 436 |
| Ni$_{0.6}$Cu$_{0.4}$Fe$_2$O$_4$ | 401 |
| Ni$_{0.4}$Cu$_{0.6}$Fe$_2$O$_4$ | 336 |
| Ni$_{0.2}$Cu$_{0.8}$Fe$_2$O$_4$ | 285 |

Unter der Curie-Temperatur Tc der magnetisierbaren und/oder magnetischen Partikel wird im Zusammenhang mit der Erfindung die Curie-Temperatur Tc mindestens einer Komponente der magnetisierbaren und/oder magnetischen Partikel verstanden. Die magnetisierbaren und/oder magnetischen Partikel können gleichzeitig verschiedene Komponenten mit verschiedenen Curie-Temperaturen Tc enthalten. In so einem Fall ist die Curie-Temperatur Tc gemeint, welche im Verlauf des Verfahrens von der Temperatur Tp der magnetisierbaren und/oder magnetischen Partikel unterschritten oder überschritten wird oder unterschritten oder überschritten werden kann. Bevorzugt sind die magnetisierbaren und/oder magnetischen Partikel phasenrein, so dass eine resultierende Curie-Temperatur Tc homogen über die magnetisierbaren und/oder magnetischen Partikel verteilt ist. Eine Homogenisierung kann beispielsweise bei der Partikelherstellung durch eine abschließende Hochtemperaturbehandlung bei circa 1000°C mit einem anschließenden Kälteschock erreicht werden.

Eine Bewegung der magnetisierbaren und/oder magnetischen Partikel in den Bilanzraum hinein oder aus dem Bilanzraum heraus kann durch unterschiedliche Kräfte bewirkt werden. In bevorzugten Ausführungsformen werden die magnetisierbaren und/oder magnetischen Partikel durch das strömende Fluid, die Schwerkraft oder das Magnetfeld transportiert.

In einer Ausführungsform werden die magnetisierbaren und/oder magnetischen Partikel durch das strömende Fluid, das gasförmig oder flüssig sein kann, transportiert. Dabei ist die Bewegung der magnetisierbaren und/oder magnetischen Partikel in Strömungsrichtung des Fluids gerichtet und wird durch die Reibung zwischen Fluid und Oberfläche der magnetisierbaren und/oder magnetischen Partikel verursacht. Hierbei können die magnetisierbaren und/oder magnetischen Partikel mit dem Fluid und/oder durch eine separate Zuführung in den Bilanzraum eintreten, wo sie dann aufgrund des Magnetfeldes lokalisiert werden, wenn die magnetisierbaren und/oder magnetischen Partikel ferromagnetische Eigenschaften aufweisen. Ebenso können magnetisierbare und/oder magnetische Partikel mit einem strömenden Fluid aus dem Bilanzraum, in dem das Magnetfeld herrscht, ausgetragen werden, wenn sie paramagnetische Eigenschaften aufweisen.

In einer weiteren Ausführungsform herrscht ein Magnetfeld nur außerhalb oder am Rand des Bilanzraums. Das Magnetfeld außerhalb des Bilanzraums kann dazu eingesetzt werden, magnetisierbare und/oder magnetische Partikel mit ferromagnetischen Eigenschaften aus dem Bilanzraum zu entfernen.

Magnetisierbare und/oder magnetische Partikel mit ferromagnetischen Eigenschaften können in einer alternativen Ausführungsform auch aus dem Bilanzraum entfernt werden, indem in dem Bilanzraum ein bewegtes Magnetfeld zur Förderung der magnetisierbaren und/oder magnetischen Partikel angelegt wird. Dazu werden mehrere Spulen übereinander oder nebeneinander angeordnet. Die magnetisierbaren und/oder magnetischen Partikel werden durch das Magnetfeld aggregiert und durch Einschalten und Ausschalten benachbarter Spulen transportiert, da die benachbarten Magnetfelder überlappen und diese beim Ausschalten der zugehörigen Spule nicht sofort erlöschen.

Je nach räumlicher vertikaler Anordnung können magnetisierbare und/oder magnetische Partikel in weiteren Ausführungsformen in den Bilanzraum hinein oder aus dem Bilanzraum hinaus transportiert werden, wenn diese noch nicht oder nicht mehr unter dem Einfluss des Magnetfeldes stehen, welches sich in dem Bilanzraum oder außerhalb des Bilanzraumes befinden kann. So können beispielsweise magnetisierbare ferromagnetische Partikel mittels Schwerkraft in den Bilanzraum transportiert werden, wo sie von einem Magnetfeld lokalisiert werden oder magnetisierbare paramagnetische Partikel können aus einem Bilanzraum mittels Schwerkraft transportiert werden, wo sie von einem Magnetfeld lokalisiert wurden bis sich die magnetischen Eigenschaften von ferromagnetisch zu paramagnetisch zum Beispiel auf Grund von Temperaturveränderungen oder einer Änderung der Zusammensetzung geändert haben. Lokalisiert bedeutet in diesem Zusammenhang, dass das Magnetfeld die ferromagnetischen magnetisierbaren und/oder magnetischen Partikel daran hindert den Bilanzraum zu verlassen. Dies schließt eine Bewegung der ferromagnetischen magnetisierbaren und/oder magnetischen Partikel im Bilanzraum nicht aus.

In einer bevorzugten Ausführungsform sind ferromagnetische magnetisierbare und/oder magnetische Partikel durch ein Magnetfeld in dem Bilanzraum lokalisiert. Während des Verfahrens nimmt die Temperatur Tp auf Grund von im Bilanzraum ablaufenden Vorgängen, zum Beispiel chemischen exothermen Reaktionen, Adsorption oder Zugabe von Substanzen mit einer höheren Temperatur als die anfängliche Temperatur Tp, zu, bis die Temperatur Tp gleich der oder höher als die Curie-Temperatur Tc der magnetisierbaren und/oder magnetischen Partikel ist. Ist die Curie-Temperatur Tc erreicht, werden die ferromagnetischen magnetisierbaren und/oder magnetischen Partikel paramagnetisch, stehen nicht länger unter dem Einfluss des Magnetfeldes und werden aus dem Bilanzraum transportiert. Hierbei kann der Transport der magnetisierbaren und/oder magnetischen Partikel aus dem Bilanzraum dazu genutzt werden Wärme aus dem System abzuführen. Der Massentransport kann also mit dem Wärmetransport gekoppelt werden. Darüber hinaus tritt beim Übergang der magnetisierbaren und/oder magnetischen Partikel von ferromagnetischen zu paramagnetischen Eigenschaften ein magnetokalorischer Effekt auf. Die magnetischen Momente in den magnetisierbaren und/oder magnetischen Partikeln werden nicht mehr durch das Magnetfeld ausgerichtet. Es findet ein Phasenübergang zweiter Ordnung statt. Die spezifische Wärmekapazität, das heißt die zweite Ableitung der spezifischen Enthalpie nach der Temperatur, der magnetisierbaren und/oder magnetischen Partikel ändert sich sprunghaft und es kommt zu einer plötzlichen Abkühlung. Dies ist bezüglich der Temperaturführung mit dem Ziel der Wärmeabfuhr in dieser Ausführungsform vorteilhaft.

In einer alternativen Ausführungsform befinden sich paramagnetische magnetisierbare und/oder magnetische Partikel in dem Bilanzraum. Während des Verfahrens nimmt die Temperatur Tp, die anfänglich größer als die Curie-Temperatur Tc der magnetisierbaren und/oder magnetischen Partikel ist, ab, bis die Temperatur Tp niedriger als die Curie-Temperatur Tc ist. Dies geschieht zum Beispiel auf Grund von einer im Bilanzraum ablaufenden endothermen chemischen Reaktion oder durch Wärmeverluste über die Grenzen des Bilanzraumes durch Konvektion oder Wärmeleitung oder durch Zugabe von Substanzen mit einer Temperatur, die kleiner ist als die anfängliche Temperatur Tp. Ist die Curie-Temperatur Tc erreicht, werden die paramagnetischen magnetisierbaren und/oder magnetischen Partikel ferromagnetisch und stehen jetzt unter dem Einfluss eines Magnetfeldes, welches die nun ferromagnetischen magnetisierbaren und/oder magnetischen Partikel aus dem Bilanzraum transportiert. Der Bilanzraum kann dabei so gewählt sein, dass die nun ferromagnetischen magnetisierbaren und/oder magnetischen Partikel in eine in der Wirbelschicht befindliche, einem Wärmeeintrag dienenden Zone transportiert werden. Das Magnetfeld kann entweder ein statisches außerhalb des Bilanzraumes herrschendes Magnetfeld oder ein bewegtes im Bilanzraum herrschendes Magnetfeld sein. Hierbei kann wiederum ein Transport von magnetisierbaren und/oder magnetischen Partikeln in den Bilanzraum mit einer Temperatur, die höher ist als die Temperatur $T_p$ der magnetisierbaren und/oder magnetischen Partikel im Bilanzraum, dazu genutzt werden um dem System Wärme zuzuführen. Als Ausgleich und zur Reduzierung der zu erwärmenden Masse im Bilanzraum können dann wie hier beschrieben abgekühlte magnetisierbare und/oder magnetische Partikel aus dem Bilanzraum entfernt werden. Im beanspruchten Verfahren wird in dem Bilanzraum eine chemische Reaktion durchgeführt. Unter einer chemischen Reaktion wird eine Reaktion verstanden, bei der eine Stoffumwandlung stattfindet. Die chemische Reaktion kann eine exotherme oder eine endotherme Reaktion sein.

Handelt es sich bei der chemischen Reaktion um eine exotherme Reaktion, so wird Reaktionsenergie frei, welche zur Erhöhung der Temperatur $T_p$ beitragen kann. In einer bevorzugten Ausführungsform ist die durch die exotherme Reaktion hervorgerufene Temperaturerhöhung im Bilanzraum dadurch begrenzt, dass magnetisierbare und/oder magnetische Partikel, die paramagnetische Eigenschaften aufweisen, da ihre Curie-Temperatur $T_c$ überschritten ist, wie oben beschrieben aus dem Bilanzraum transportiert werden.

In einer bevorzugten Ausführungsform ist die chemische Reaktion eine stark exotherme Reaktion. Als stark exotherm werden Reaktionen bezeichnet, deren Betrag der Reaktionsenthalpie größer als 200 kJ/mol, bevorzugt größer als 300 kJ/mol und insbesondere größer als 350 kJ/mol ist.

Das erfindungsgemäße Verfahren kann insbesondere vorteilhaft zur Temperaturführung von heterogen katalysierten exothermen Reaktionen eingesetzt werden, da dadurch eine ausreichende, dem Reaktionsverlauf angepasste Wärmeabfuhr sichergestellt werden kann. Eine unzureichende Wärmeabfuhr kann zur Bildung von hot spots, also lokalen Temperaturerhöhungen, unerwünschten Temperaturprofilen und zu einem Durchgehen, also einer Überhitzung des Reaktors, führen. Diese Erscheinungen verstärken beispielsweise eine Bildung von Nebenprodukten, eine Deaktivierung des Katalysators oder Verkokung. Weiterhin ist die Gewährleistung der Betriebssicherheit eine Grundvoraussetzung für die Durchführung von exothermen Reaktionen, die von einer kontrollierten Temperaturführung abhängt.

Handelt es sich bei der chemischen Reaktion um eine endotherme Reaktion, so wird dem System durch das Fortschreiten der Reaktion Wärme entzogen, was in einer Abnahme der Temperatur $T_p$ resultieren kann.

In einer bevorzugten Ausführungsform ist die Curie-Temperatur $T_c$ der magnetisierbaren und/oder magnetischen Partikel auf die spezifische chemische im Bilanzraum durchgeführte Reaktion, welche unter anderem durch mindestens ein beteiligtes Edukt, mindestens ein beteiligtes Produkt und eine Reaktionstemperatur $T_r$ bestimmt wird, abgestimmt. Die Reaktionstemperatur $T_r$ ist die Temperatur, bei der die chemische Reaktion durchgeführt wird. Die Reaktionstemperatur $T_r$ wird in bevorzugter Weise nach einer Optimierung unter verfahrenstechnischen Gesichtspunkten gewählt. Die Curie-Temperatur $T_c$ der magnetisierbaren und/oder magnetischen Partikel beträgt bevorzugt zwischen 100°C und 900°C, insbesondere bevorzugt zwischen 350°C und 400°C. Besonders bevorzugt weicht die Curie-Temperatur $T_c$ der magnetisierbaren und/oder magnetischen Partikel nicht mehr als 150 K, insbesondere nicht mehr als 50 K, und optimalerweise nicht mehr 20 K von der Reaktionstemperatur $T_r$ der chemischen Reaktion ab. Sind in den magnetisierbaren und/oder magnetischen Partikeln verschiedene Komponenten mit verschiedenen Curie-Temperaturen enthalten, so ist mit der Curie-Temperatur $T_c$ der magnetisierbaren und/oder magnetischen Partikel die Curie-Temperatur $T_c$ gemeint, welche am nächsten bei der Reaktionstemperatur $T_r$ liegt.

In einer weiteren Ausführungsform katalysieren die magnetisierbaren und/oder magnetischen Partikel die chemische Reaktion oder enthalten ein bezüglich der chemischen Reaktion katalytisch aktives Material. Werden in diesem Fall die magnetisierbaren und/oder magnetischen Partikel aus dem Bilanzraum transportiert, so wird die dort durchgeführte chemische Reaktion verlangsamt oder gestoppt. Ist die chemische Reaktion gleichzeitig eine exotherme Reaktion, so wird auf diese Weise die Wärmeentwicklung in dem Bilanzraum begrenzt.

Die magnetischen Eigenschaften der magnetisierbaren und/oder magnetischen Partikel können nicht nur temperaturabhängig sein, sondern auch oder alternativ von der Zusammensetzung der magnetisierbaren und/oder magnetischen Partikel abhängen. So können beispielsweise unterschiedliche Oxidationsstufen mindestens einer Komponente der magnetisierbaren und/oder magnetischen Partikel unterschiedliche magnetische Eigenschaften aufweisen. Die chemische Reaktion kann die Oxidationsstufen mindestens einer Komponente der magnetisierbaren und/oder magnetischen Partikel verändern.

Katalysieren die magnetisierbaren und/oder magnetischen Partikel die chemische Reaktion oder enthalten sie ein bezüglich der chemischen Reaktion katalytisch aktives Material und wird durch Katalysatordeaktivierung während der chemischen Reaktion beziehungsweise durch Katalysatoraktivierung während einer Regeneration die Zusammensetzung der magnetisierbaren und/oder magnetischen Partikel so geändert, dass sich auch die magnetischen Eigenschaften der Partikel ändern, so entscheidet das Ausmaß an Katalysatordeaktivierung beziehungsweise Katalysatoraktivierung über den Aufenthaltsort der magnetisierbaren und/oder magnetischen Partikel, welcher im Bilanzraum oder außerhalb des Bilanzraums sein kann. Das System kann sich in dieser Ausführungsform selbst regulieren, indem deaktivierter Katalysator aus dem Bilanzraum transportiert und/oder aktivierter Katalysator in den Bilanzraum transportiert wird. Beispielsweise besitzt ein Katalysator in der Styrolsynthese in deaktivierter Form andere magnetische Eigenschaften als derselbe Katalysator in nicht deaktivierter Form wie beispielsweise Eisen in unterschiedlichen Oxidationsstufen. In einem weiteren Beispiel können in einer ersten Verfahrensstufe organische Schwefelverbindungen und Schwefelwasserstoff wie folgt gespalten werden:

$$FeS + R\text{-}SH \rightarrow FeS_2 + R\text{-}H$$

$$FeS + H_2S - FeS_2 + H_2.$$

Die erste Verfahrensstufe kann zum Beispiel in einer Wirbelschicht oder in einem anderen, feststofffförderndem Reaktor durchgeführt werden. Vom festen $FeS_2$ kann anschließend eine Flüssigphase beziehungsweise eine Gasphase, die ein entschwefeltes Produkt enthält, abgetrennt und das $FeS_2$ in einen Regenerator gefördert werden. In dem Regenerator kann bei hohen Temperaturen eine Rückspaltung zu FeS erfolgen. Elementarer Schwefel S kann abgetrennt und FeS in die erste Verfahrensstufe zurückgeführt werden.

In einer alternativen Ausführungsform sind die magnetisierbaren und/oder magnetischen Partikel Edukt oder Produkt der chemischen Reaktion, wobei sich die magnetischen Eigenschaften des Eduktes von den magnetischen Eigenschaften des entsprechenden Produktes unterscheiden und jeweils eines ferromagnetisch und das andere paramagnetisch ist. Die Änderung der magnetischen Eigenschaften der magnetisierbaren und/oder magnetischen Partikel kann auch in diesem Fall mit der Änderung der Oxidationsstufe durch die chemische Reaktion mindestens einer Komponente der magnetisierbaren und/oder magnetischen Partikel verknüpft sein. Auf diese Weise kann zum Beispiel ein ferromagnetisches Edukt durch das Magnetfeld in dem Bilanzraum lokalisiert werden, bis es zum paramagnetischen Produkt umgesetzt und dann als Produkt auf Grund von oben genannten Kräften aus dem Bilanzraum transportiert wird. Auf diese Weise wird die Verweilzeit des Eduktes im Bilanzraum dahingehend optimiert, dass das Edukt so lange im Bilanzraum verbleibt bis es zum Produkt umgesetzt ist.

Alternativ kann ein paramagnetisches Edukt im Bilanzraum zu einem ferromagnetischen Produkt umgesetzt werden, das dann durch ein Magnetfeld außerhalb des Bilanzraumes oder durch ein bewegtes Magnetfeld im Bilanzraum aus dem Bilanzraum transportiert wird. Ein Vorteil dieser Ausführungsform ist, dass die Reaktion direkt mit einem Trennverfahren kombiniert wird und eine häufig benötigte nachträgliche Trennung von Produkt und nicht umgesetztem Edukt überflüssig wird. Darüber hinaus kann der Umsatz erhöht werden, indem ein paramagnetisches Edukt so lange im Bilanzraum verbleibt bis es umgesetzt ist und erst dann unter Einfluss des Magnetfeldes steht und nicht vorzeitig noch als Edukt aus dem Bilanzraum ausgetragen wird. Hierbei wir davon ausgegangen, dass im Bilanzraum reaktionsfördernde Bedingungen hinsichtlich zum Beispiel der Temperatur und Anwesenheit eines Katalysators herrschen.

In einer weiteren Ausführungsform werden die magnetisierbaren und/oder magnetischen Partikel nach dem Transport aus dem Bilanzraum gekühlt oder erwärmt und/oder regeneriert und anschließend gegebenenfalls in den Bilanzraum zurückgeführt. Somit können die magnetisierbaren und/oder magnetischen Partikel als Wärmetauschermedium dienen oder die Verfahrensstabilität oder Katalysatorausnutzung erhöhen, indem deaktivierter Katalysator selektiv aus dem Bilanzraum entfernt und aktivierter Katalysator dem Bilanzraum zugeführt werden kann.

Im Fall der Durchführung einer exothermen Reaktion besteht das System bevorzugt aus einem magnetisch unterstützen Reaktor (MER) und einem Kühler. Die Curie-Temperatur Tc der magnetisierbaren und/oder magnetischen Partikel, die bevorzugt einen Katalysator der exothermen Reaktion enthalten, wird bevorzugt entsprechend der gewünschten Reaktionstemperatur Tr mittels entsprechendem Herstellungsverfahren eingestellt. Magnetisierbare und/oder magnetische Partikel, die im MER, wo sie vom Magnetfeld lokalisiert werden, auf eine Temperatur Tp aufgeheizt werden, die höher als ihre Curie-Temperatur Tc ist, verlieren ihre magnetischen Eigenschaften und werden zum Kühler befördert. Nach Abkühlung auf eine Temperatur Tp, die niedriger als ihre Curie-Temperatur Tc ist, verhalten sich die magnetisierbaren und/oder magnetischen Partikel wieder magnetisch und werden wieder dem Reaktor zugeführt. Durch diese Temperaturkontrolle exothermer Reaktionen können sogenannte hot-spots und ein Durchgehen von Reaktionen vermieden werden, der Wärmetransport und die Selektivität werden verbessert und die Katalysatordeaktivierung wird reduziert.

Zur Kühlung oder Erwärmung können die magnetisierbaren und/oder magnetischen Partikel einem Bereich zugeführt werden, der entsprechend temperiert ist oder sie können in Kontakt mit einer Wärmetauschervorrichtung gebracht werden. Für die Zuführung der Partikel zur Temperierung kann je nach Reaktorkonzept die Strömung des Fluids oder die Magnetkraft genutzt werden.

In einer weiteren Ausführungsform dient das Verfahren einer Absicherung eines Reaktors, in dem eine exotherme Reaktion durchgeführt wird. Dabei wird die exotherme Reaktion bei einer Regelreaktionstemperatur Trr durchgeführt, die niedriger ist als eine Grenztemperatur Tg, die Curie-Temperatur Tc der magnetisierbaren und/oder magnetischen Partikel ist so eingestellt, dass die Curie-Temperatur Tc der magnetisierbaren und/oder magnetischen Partikel größer ist als die Reaktionstemperatur Tr und kleiner ist als die Grenztemperatur Tg und eine Differenz zwischen der Curie-Temperatur Tc der magnetisierbaren und/oder magnetischen Partikel und der Grenztemperatur Tg nicht mehr als 20°C beträgt und sich die magnetischen Eigenschaften der magnetisierbaren und/oder magnetischen Partikel nur ändern, wenn die Reaktionstemperatur Tr, die zu Anfang gleich der Regelreaktionstemperatur Trr ist, zunimmt und dadurch die Temperatur Tp der magnetisierbaren und/oder magnetischen Partikel zunimmt und die Temperatur Tp der magnetisierbaren und/oder magnetischen Partikel die Curie-Temperatur Tc überschreitet. Bevorzugt katalysieren dabei die magnetisierbaren und/oder magnetischen Partikel die exotherme Reaktion oder enthalten ein katalytisch aktives Material. Bei planmäßiger Durchführung der exothermen Reaktion bei der Regelreaktionstemperatur Trr bleiben die magnetischen Eigenschaften der magnetisierbaren und/oder magnetischen Partikel unverändert. Die Regelreaktionstemperatur Trr ist die Temperatur, bei der die Reaktion gemäß der Auslegung des Verfahrens durchgeführt wird. Die Curie-Temperatur Tc der magnetischen Partikel ist so auf die Reaktionsbedingungen und die eingesetzten Apparate abgestimmt, insbesondere auf die Regelreaktionstemperatur Trr und auf die Grenztemperatur Tg, dass im Falle von möglichen Zwischenfällen, die zu einer ungeplanten Temperaturerhöhung im Bilanzraum führen, der Katalysator, aufgrund der Änderung der magnetischen Eigenschaften der magnetisierbaren und/oder magnetischen Partikel, aus dem Bilanzraum ausgetragen wird. Auf diese Weise kann eine Überhitzung des Reaktors, welche auch als Durchgehen des Reaktors bezeichnet wird, verhindert werden. Dies Verfahren zur Absicherung einer exothermen Reaktion stellt sicher, dass die Wärmeentwicklung reduziert oder gestoppt wird und gleichzeitig Wärme gekoppelt an Massentransport aus dem Bilanzraum abgeführt wird, indem der Katalysator von den Edukten getrennt wird, sobald die aktuelle Reaktionstemperatur Tr und damit auch die Temperatur Tp der magnetisierbaren und/oder magnetischen Partikel die Curie-Temperatur Tc der magnetisierbaren und/oder magnetischen Partikel überschreitet. Bevorzugt ist die Curie-Temperatur Tc der magnetisierbaren und/oder magnetischen Partikel dabei so eingestellt, dass sie an der Grenztemperatur Tg des Reaktionssystems orientiert ist. Die Grenztemperatur Tg ist die Temperatur, ab welcher Schäden durch Überhitzung zu erwarten wären und die magnetisierbaren und/oder magnetischen Partikel aus dem Bilanzraum beziehungsweise Reaktor ausgetragen werden sollen. Die Grenztemperatur Tg ist systemspezifisch und unter anderem abhängig von dem Material des Reaktors, das die Druckfestigkeit und Hitzebeständigkeit des Reaktors bestimmt, sowie von möglichen Nebenreaktionen, die bei erhöhten Temperaturen ablaufen könnten und vermieden werden sollen. Die Differenz zwischen der Curie-Temperatur Tc der magnetisierbaren und/oder magnetischen Partikel und der Grenztemperatur Tg beträgt bevorzugt nicht mehr als 100°C, mehr bevorzugt nicht mehr als 50°C, besonders bevorzugt nicht mehr als 20°C und insbesondere bevorzugt nicht mehr als 10°C. Die Regelreaktionstemperatur Trr beträgt bevorzugt zwischen 100° und 900°C, mehr bevorzugt zwischen 200°C und 600°C und besonders bevorzugt zwischen 250°C und 350°C. In dieser Ausführungsform läuft das erfindungsgemäße Verfahren unter planmäßigen Verfahrensbedingungen nicht ab, es sind aber die nötigen Vorkehrungen getroffen worden, so dass das erfindungsgemäße Verfahren ablaufen kann, wenn es zu einem unplanmäßigen Temperaturanstieg im Reaktor kommt.

Der Bilanzraum kann Teil eines jeden dem Fachmann bekannten Reaktortyps sein. In einer bevorzugten Ausführungsform befindet sich der Bilanzraum in einem Festbettreaktor, in einem expanded-bed-Reaktor, der auch expandierter Bettreaktor oder Fließbettreaktor genannt wird und im Vergleich zum Festbettreaktor eine Bewegung der Feststoffe im Bett ermöglicht, in einem Suspensionsreaktor, in einem Wirbelschichtreaktor oder in einem Reaktor, der eine magnetfeldunterstützte Wirbelschicht umfasst. Insbesondere bevorzugt sind ein Wirbelschichtreaktor und ein Reaktor, der eine magnetisch unterstütze Wirbelschicht umfasst.

Die Anwendung des erfindungsgemäßen Verfahrens ist für viele verschiedenartige Reaktionssysteme denkbar, wie die Aufreinigung von Caprolactam, die Herstellung von Ethylen aus Acetylen, die Hochtemperatur-Fischer-Tropsch-Synthese, die Ammoniumsynthese, die Rauchgasentschwefelung, die Herstellung von Biodiesel, die Dehalogenierung in der Abwasseraufbereitung, die Synthesegasherstellung mit der Trennung von Wasserstoff und Kohlenstoffdioxid, die Oligomerisierung von Olefinen, die Herstellung von Wasserstoff aus Methan, die Reformierung von Kohlenwasserstoffen, die Trennung von Olefinen und Paraffinen, die katalytische Reduktion von Stickstoffmonoxid, die Methanisierung von Kohlenmonoxid und die Synthese von Kohlensäurediphenylester. Andere Anwendungsbereiche sind weitere Prozesse der Petrochemie und Umwelttechnik, ebenso wie bioverfahrenstechnische Prozesse, bei denen Enzyme auf magnetisierbaren und/oder magnetischen Partikeln immobilisiert sind, und andere Reaktionen und Trennprozesse wie Chromatographie und Trocknung.

In einer bevorzugten Ausführungsform ist die chemische Reaktion eine Hydrierung, bevorzugt eine Herstellung eines aromatischen Amins und insbesondere bevorzugt eine Umsetzung von Nitrobenzol zu Anilin. Für die Herstellung von Anilin stellt das erfindungsgemäße Verfahren eine Alternative oder Ergänzung zum Beispiel zu einem in die Wirbelschicht integrierten Wärmeübertrager dar, welcher der Wärmeabfuhr aus der exothermen Reaktion dient.

Kurze Beschreibung der Zeichnungen

Ausführungsbeispiele der Erfindung sind in den Zeichnungen dargestellt und werden in der nachfolgenden Beschreibung näher erläutert. Es zeigen

Figur 1    eine schematische Darstellung einer exothermen Reaktion in einer magnetfeldunterstützten Wirbelschicht mit selbstregulierender Temperaturführung,

Figur 2      eine schematische Darstellung einer exothermen Reaktion in einer magnetfeldunterstützten Wirbelschicht mit selbstregulierender Temperaturführung und Rückführung magnetisierbarer und/oder magnetischer Partikel,

Figur 3      eine schematische Darstellung einer exothermen Reaktion in einem Batchreaktor mit alternierendem Magnetfeld und selbstregulierender Temperaturführung,

Figur 4      eine schematische Darstellung einer endothermen Reaktion in einer Wirbelschicht mit selbstregulierender Temperaturführung,

Figur 5      eine schematische Darstellung einer endothermen Reaktion in einer Wirbelschicht mit selbstregulierender Temperaturführung und seitlich angeordnetem Heizelement,

Figur 6      eine schematische Darstellung einer endothermen Reaktion in einem Batchreaktor mit selbstregulierender Temperaturführung,

Figur 7      eine schematische Darstellung einer endothermen Reaktion in einem Batchreaktor mit selbstregulierender Temperaturführung und magnetischer Förderung,

Figur 8      Umsätze von Nitrobenzol in Anwesenheit von magnetisierbaren und/oder magnetischen Partikeln und

Figur 9      Temperaturverläufe bei der Erwärmung von magnetisierbaren und/oder magnetischen Partikeln.

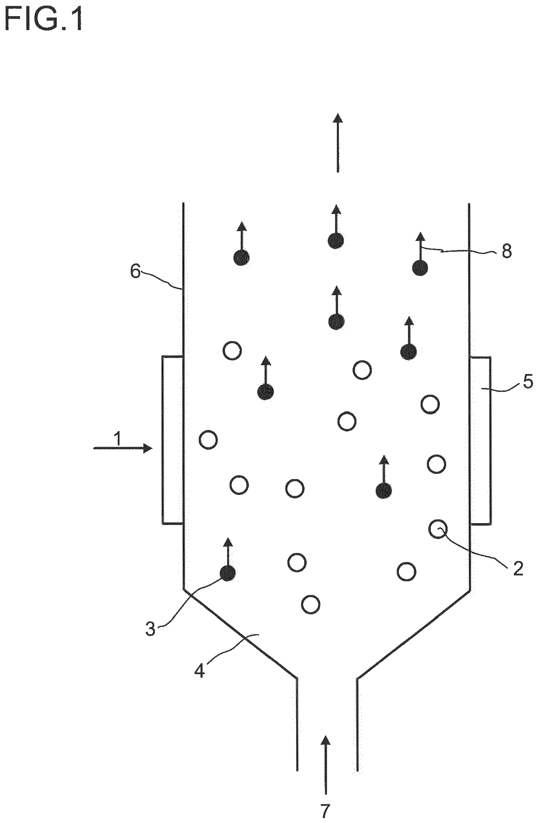

Figur 1 zeigt eine schematische Darstellung einer exothermen Reaktion in einer magnetfeldunterstützten Wirbelschicht mit selbstregulierender Temperaturführung.

In Figur 1 ist eine erste Wirbelschicht 1 dargestellt, in der eine exotherme Reaktion durchgeführt wird. Dazu strömt eine fluide Phase 4, in der sich mindestens ein Edukt befindet, durch die erste Wirbelschicht 1, die durch eine Reaktorwand 6 begrenzt ist. In der ersten Wirbelschicht 1 sind magnetisierbare und/oder magnetische Partikel fluidisiert. Die magnetisierbaren und/oder magnetischen Partikel weisen eine katalytische Aktivität bezüglich der in der ersten Wirbelschicht 1 durchgeführten exothermen Reaktion auf. Durch mindestens eine Magnetspule 5 wird im Bereich der ersten Wirbelschicht 1 ein statisches Magnetfeld erzeugt, welches die Wirbelschicht stabilisiert und eine Kraft auf magnetische Partikel ausübt, die der Strömungsrichtung 7 der fluiden Phase 4 entgegengerichtet ist . Ein erster Teil 2 der magnetisierbaren und/oder magnetischen Partikel weist eine Temperatur $Tp2$ auf, die geringer ist als ihre Curie-Temperatur $Tc$. Der erste Teil 2 der magnetisierbaren und/oder magnetischen Partikel steht also unter Einfluss des angelegten Magnetfeldes. Die Bewegung des ersten Teils 2 der magnetisierbaren und/oder magnetischen Partikel in der ersten Wirbelschicht 1 ist nicht gerichtet und der erste Teil 2 der magnetisierbaren und/oder magnetischen Partikel verbleibt in der ersten Wirbelschicht 1, ist in der ersten Wirbelschicht 1 lokalisiert. Ein zweiter Teil 3 der magnetisierbaren und/oder magnetischen Partikel weist eine Temperatur $Tp3$ auf, die höher ist als ihre Curie-Temperatur $Tc$. Der zweite Teil 3 der magnetisierbaren und/oder magnetischen Partikel wird also nicht vom Magnetfeld beeinflusst. Die vom Magnetfeld erzeugte Kraft, die der Strömungsrichtung 7 entgegengerichtet ist, entfällt für den zweiten Teil 3 der magnetisierbaren und/oder magnetischen Partikel, der daher in Strömungsrichtung 8 aus der ersten Wirbelschicht 1 ausgetragen wird. Die Temperaturführung in der ersten Wirbelschicht 1 ist selbstregulierend. Durch den Fortschritt der heterogen katalysierten exothermen Reaktion steigt die Temperatur in der Wirbelschicht an und somit auch die Temperatur $Tp$ der magnetisierbaren und/oder magnetischen Partikel, so dass einzelne Partikel vom ersten Teil 2 der magnetisierbaren und/oder magnetischen Partikel in den zweiten Teil 3 der magnetisierbaren und/oder magnetischen Partikel übergehen, den Einfluss des Magnetfeldes verlieren, von der fluiden Phase 4 mitgerissen und aus der ersten Wirbelschicht 1 ausgetragen werden. Auf diese Weise nimmt die Menge an Katalysator in der ersten Wirbelschicht 1 ab und damit auch die Reaktionsgeschwindigkeit und die Temperaturzuwachsrate, welche darüber hinaus auch durch das Zuströmen der fluiden Phase 4 beeinflusst und von außen reguliert werden kann. Es besteht die Möglichkeit Partikel des zweiten Teils 3 der magnetisierbaren und/oder magnetischen Partikel, die aus der ersten Wirbelschicht 1 ausgetragen wurden, abzukühlen und in die Wirbelschicht zurückzuführen.

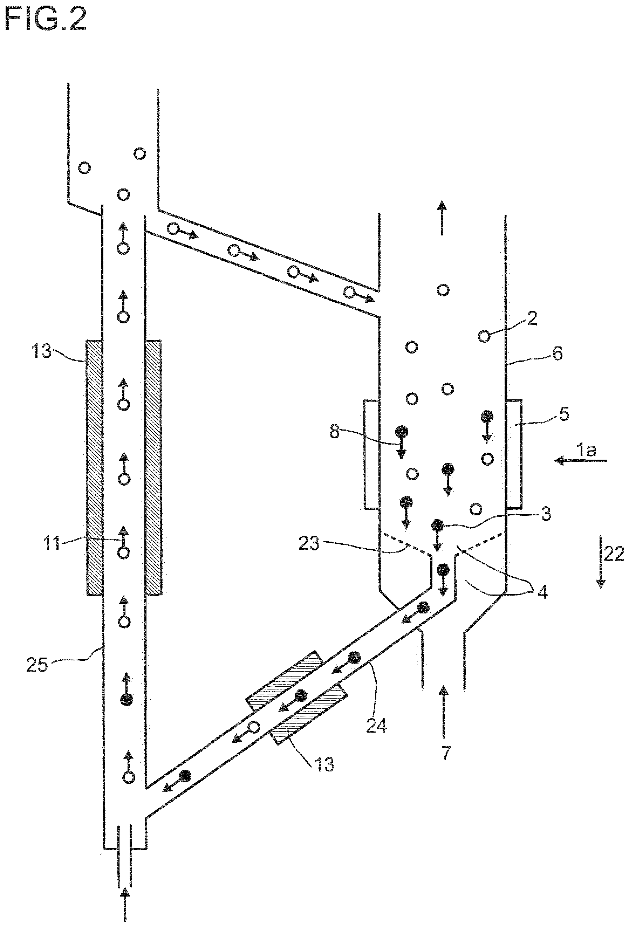

Figur 2 zeigt eine schematische Darstellung einer exothermen Reaktion in einer magnetfeldunterstützten Wirbelschicht mit selbstregulierender Temperaturführung und Rückführung magnetisierbarer und/oder magnetischer Partikel.

Eine in der Figur 2 gezeigte zweite Wirbelschicht 1a unterscheidet sich von der in Figur 1 dargestellten ersten Wirbelschicht 1 bezüglich der Strömungsgeschwindigkeit der fluiden Phase 4 durch die Wirbelschicht. Die Strömungsgeschwindigkeit der fluiden Phase 4 in Figur 2 ist geringer, so dass der zweite Teil 3 der magnetisierbaren und/oder magnetischen Partikel, der nicht unter Einfluss des Magnetfeldes steht, der Schwerkraft folgt und absinkt. Außerdem wird durch die mindestens eine Magnetspule 5 ein Magnetfeld erzeugt, welches auf magnetische Partikel eine Kraft ausübt, die in Richtung der Strömungsrichtung 7 der fluiden Phase 4 gerichtet ist. Wird die Curie-Temperatur Tc einzelner magnetisierbarer und/oder magnetischer Partikel überschritten, so verlassen diese die zweite Wirbelschicht 1a durch ein Fallrohr 24. Vereinzelt könnten auch magnetisierbare und/oder magnetische Partikel des ersten Teils 2 in das Fallrohr eintreten. Der Anteil von Partikeln des zweiten Teils 3 im Fallrohr ist aber im Allgemeinen größer als der Anteil von Partikeln des ersten Teils 2. Der Umfang des Fallrohrs 24 ist beim Eintritt des Fallrohrs 24 in die zweite Wirbelschicht 1a von einem Gitter 23 umgeben, das den Umfang des Fallrohrs 24 mit der Reaktorwand 6 verbindet. Die fluide Phase 4 kann durch das Gitter 23 strömen, wohingegen die magnetisierbaren und/oder magnetischen Partikel nicht durch das Gitter hindurchtreten können und die magnetisierbaren und/oder magnetischen Partikel, welche sich in ihrer Strömungsrichtung 8 nach unten bewegen, werden zum Fallrohr 24 hingeführt. Im Fallrohr 24 werden die magnetisierbaren und/oder magnetischen Partikel des zweiten Teils 3 der magnetisierbaren und/oder magnetischen Partikel an mindestens einem Kühlmantel 13 vorbeigeführt, so dass diese abkühlen, ihre Curie-Temperatur Tc unterschritten wird und sie in den ersten Teil 2 der magnetisierbaren und/oder magnetischen Partikel übergehen. Die abgekühlten magnetisierbaren und/oder magnetischen Partikel werden über ein Steigrohr 25 der zweiten Wirbelschicht 1a wieder zugeführt, wo sie wiederum unter Einfluss des Magnetfeldes stehen bis sie wie bezüglich Figur 1 beschrieben wieder auf eine Temperatur oberhalb ihrer Curie-Temperatur Tc durch die in der zweiten Wirbelschicht 1a durchgeführte heterogen katalysierte exotherme Reaktion erwärmt werden.

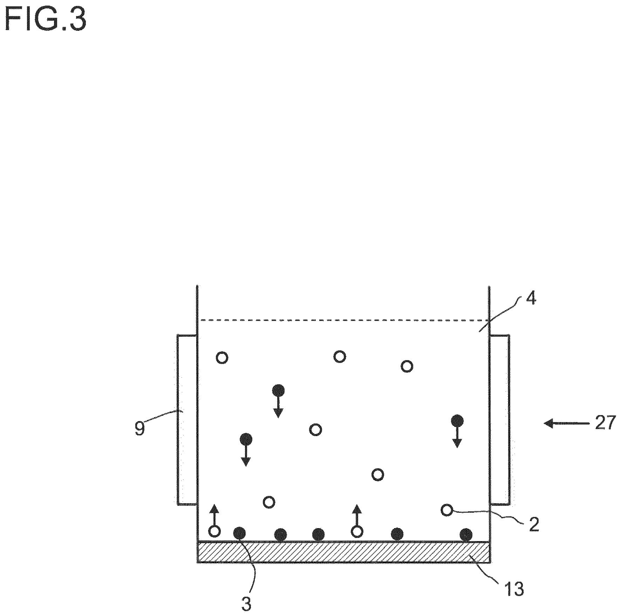

Figur 3 zeigt eine schematische Darstellung einer exothermen Reaktion in einem Batchreaktor mit alternierendem Magnetfeld und selbstregulierender Temperaturführung.

Gemäß Figur 3 wird in einem ersten Batchreaktor 27 eine exotherme Reaktion in einer fluiden Phase 4 durchgeführt, in der magnetisierbare und/oder magnetische Partikel dispergiert sind. Mindestens eine Magnetspule 9 erzeugt ein alternierendes Magnetfeld, wodurch magnetische Partikel bewegt werden und für eine Durchmischung im ersten Batchreaktor 27 gesorgt wird. Ein erster Teil 2 der magnetisierbaren und/oder magnetischen Partikel, dessen Temperatur Tp2 niedriger als die Curie-Temperatur Tc ist, steht unter Einfluss des alternierenden Magnetfeldes und wird in der fluiden Phase verwirbelt. Durch den Fortschritt der exothermen Reaktion nimmt die Temperatur im ersten Batchreaktor 27 und die Temperatur der magnetisierbaren und/oder magnetischen Partikel zu, so dass einzelne magnetisierbare und/oder magnetische Partikel des ersten Teils 2 auf eine Temperatur Tp3 erwärmt werden, die höher als ihre Curie-Temperatur Tc ist und somit in den zweiten Teil 3 der magnetisierbaren und/oder magnetischen Partikel übergehen. Der zweite Teil 3 der magnetisierbaren und/oder magnetischen Partikel steht nicht unter dem Einfluss des alternierenden Magnetfelds und sinkt nach unten. Am Boden des ersten Batchreaktors 27 befindet sich ein Kühlmantel 13, mit dem sedimentierte Partikel des zweiten Teils 3 der magnetisierbaren und/oder magnetischen Partikel in Kontakt kommen und auf eine Temperatur Tp2, die niedriger ist als ihre Curie-Temperatur Tc, abgekühlt werden. Nun stehen diese abgekühlten Partikel wieder unter Einfluss des alternierenden Magnetfeldes und werden wieder in der fluiden Phase 4 verwirbelt. Dadurch werden magnetisierbare und/oder magnetische Partikel des zweiten Teils 3 selbstregulierend dem Kühlmantel 13 zugeführt und abgekühlt. Magnetisierbare und/oder magnetische Partikel des ersten Teils 2 werden selbstregulierend vom Kühlmantel 13 entfernt, sobald ihre Temperatur die Curie-Temperatur Tc unterschreitet.

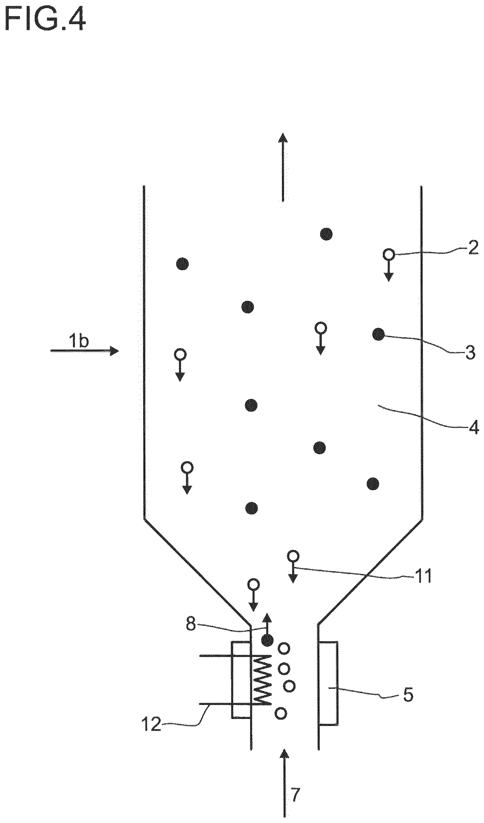

Figur 4 zeigt eine schematische Darstellung einer endothermen Reaktion in einer Wirbelschicht mit selbstregulierender Temperaturführung.

In Figur 4 wird in einer dritten Wirbelschicht 1b eine endotherme Reaktion durchgeführt. An der Zuführung der fluiden Phase 4 unterhalb der dritten Wirbelschicht 1b sind ein Heizelement 12 und mindestens eine Magnetspule 5 angeordnet. In der dritten Wirbelschicht 1b ist zunächst ein zweiter Teil 3 an magnetisierbaren und/oder magnetischen Partikeln enthalten, deren Temperatur Tp3 höher als ihre Curie-Temperatur Tc ist. Eine Zuführung der fluiden Phase 4 in die dritte Wirbelschicht 1b ist bevorzugt so gewählt, dass der zweite Teil 3 der magnetisierbaren und/oder magnetischen Partikel das Heizelement 12 und die mindestens eine Magnetspule 5 dadurch nicht erreicht, dass eine Fluidgeschwindigkeit in Strömungsrichtung 7 in einem Querschnitt auf Höhe des Heizelements 12 und der mindestens einen Magnetspule 5 an einem Eintritt der dritten Wirbelschicht 1b eine Austragsgeschwindigkeit des zweiten Teils 3 der magnetisierbaren und/oder magnetischen Partikel übersteigt. Durch Fortschritt der endothermen Reaktion nimmt die Temperatur der magnetisierbaren und/oder magnetischen Partikel ab und einzelne magnetisierbare und/oder magnetische Partikel unterschreiten ihre Curie-Temperatur Tc, so dass sie in den ersten Teil 2 der magnetisierbaren und/oder magnetischen Partikel übergehen und unter dem Einfluss des Magnetfeldes stehen, welches von der mindestens einen Magnetspule 5 erzeugt wird. Der erste Teil 2 der magnetisierbaren und/oder magnetischen Partikel wird von der mindestens einen Magnetspule 5 angezogen und bewegt sich aufgrund der nun wirkenden, vom Magnetfeld zusätzlich ausgeübten Kraft auf den ersten Teil 2 der magnetisierbaren und/oder magnetischen Partikel in Richtung der mindestens einen Magnetspule 5 und des Heizelements 12, welche so angeordnet sind, dass das Magnetfeld der mindestens einen Magnetspule 5 die magnetisierbaren und/oder magnetischen Partikel des ersten Teils 2 in Kontakt mit oder in der Nähe zu dem Heizelement 12 positioniert. Die dort positionierten magnetisierbaren und/oder magnetischen Partikel werden durch das Heizelement 12 auf eine Temperatur Tp3 oberhalb ihrer Curie-Temperatur Tc erwärmt, gehen damit in den zweiten Teil 3 der magnetisierbaren und/oder magnetischen Partikel über, stehen nicht mehr unter dem Einfluss des Magnetfeldes und werden von der strömenden fluiden Phase 4 in Strömungsrichtung 7 zurück in die dritte Wirbelschicht 1b transportiert. Im Fall einer Sekundärzufuhr der fluiden Phase 4 beispielsweise auf einer halben Höhe der dritten Wirbelschicht 1b, können mehrere Zonen in der Wirbelschicht ausgebildet werden. Hier kann das erfindungsgemäße Verfahren eingesetzt werden um eine gewünschte Partikelzirkulation in den verschiedenen Zonen zu erreichen.

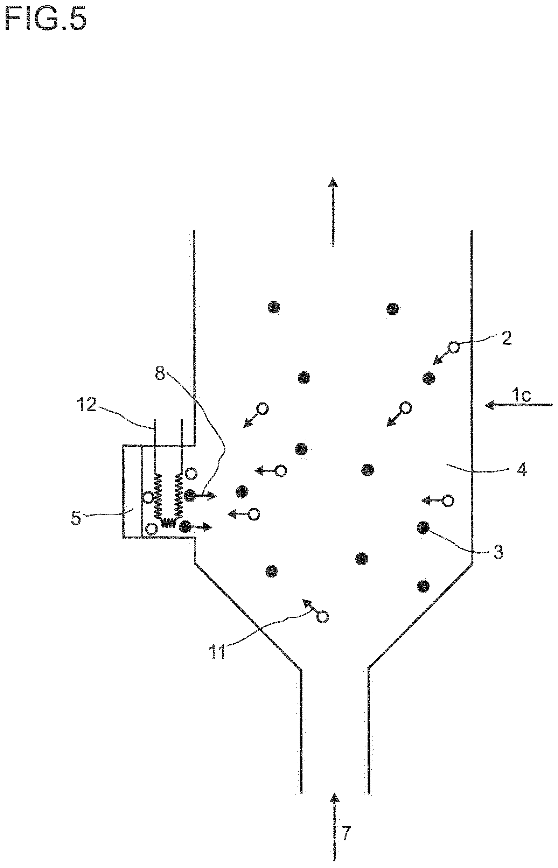

Figur 5 zeigt eine schematische Darstellung einer endothermen Reaktion in einer Wirbelschicht mit selbstregulierender Temperaturführung und seitlich angeordnetem Heizelement.

Eine in Figur 5 dargestellt vierte Wirbelschicht 1c unterscheidet sich von der dritten Wirbelschicht 1b dadurch, dass das Heizelement 12 und die mindestens eine Magnetspule 5 seitlich der vierten Wirbelschicht 1c angeordnet sind. Dadurch sind die Bewegungsrichtungen 8 und 11 der magnetisierbaren und/oder magnetischen Partikel 3 und der magnetisierbaren und/oder magnetischen Partikel 2 nicht parallel zur Strömungsrichtung 7 der fluiden Phase 4. Des Weiteren können sich die magnetisierbaren und/oder magnetischen Partikel 3 in einer Ausführungsform der vierten Wirbelschicht 1c auch zufällig in Richtung der mindestens einen Magnetspule 5 bewegen, was in der oben beschriebenen Wirbelschicht 1b bevorzugt dadurch verhindert wird, dass die Fluidgeschwindigkeit im Bereich der mindestens einen Magnetspule 5 so hoch ist, dass Partikel nur unter Einfluss des Magnetfelds zur mindestens einen Magnetspule 5 und zum Heizelement 12 gelangen können.

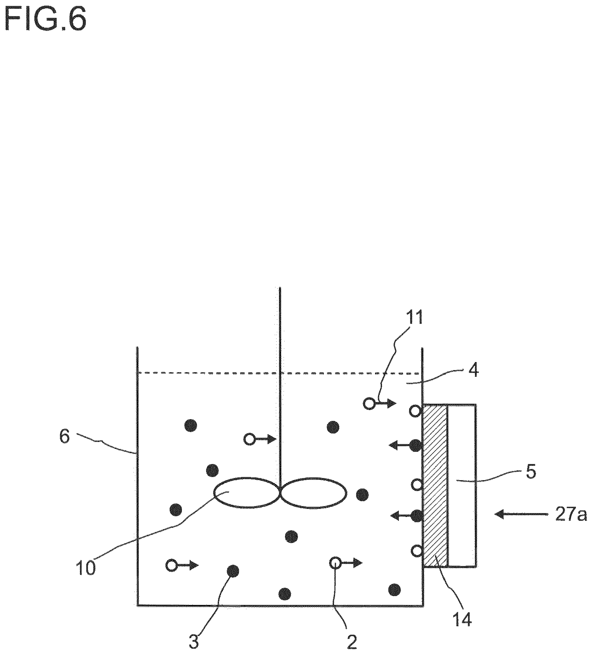

Figur 6 zeigt eine schematische Darstellung einer endothermen Reaktion in einem Batchreaktor mit selbstregulierender Temperaturführung.

Gemäß Figur 6 wird in einem zweiten Batchreaktor 27a eine endotherme Reaktion in einer fluiden Phase 4 durchgeführt, in der magnetisierbare und/oder magnetische Partikel dispergiert sind. Eine Durchmischung wird durch den Rührer 10 erreicht. Mindestens eine Magnetspule 5 und ein Heizmantel 14 sind so gemeinsam an der Reaktorwand 6 angeordnet, dass ein erster Teil 2 der magnetisierbaren und/oder magnetischen Partikel, deren Temperatur Tp2 niedriger als ihre Curie-Temperatur Tc ist, unter Einfluss des durch die mindestens eine Magnetspule 5 erzeugten Magnetfeldes steht und zum Heizmantel 14 hingezogen wird. Durch Fortschritt der endothermen Reaktion nimmt die Temperatur Tp3 der magnetisierbaren und/oder magnetischen Partikel ab und einzelne magnetisierbare und/oder magnetische Partikel des zweiten Teils 3 unterschreiten ihre Curie-Temperatur Tc, so dass sie in den ersten Teil 2 der magnetisierbaren und/oder magnetischen Partikel übergehen und unter dem Einfluss des Magnetfeldes stehen, welches von der mindestens einen Magnetspule 5 erzeugt wird. Der erste Teil 2 der magnetisierbaren und/oder magnetischen Partikel wird von der mindestens einen Magnetspule 5 angezogen und bewegt sich in Richtung der mindestens einen Magnetspule 5 und des Heizmantels 14, welche so angeordnet sind, dass das Magnetfeld der mindestens einen Magnetspule 5 die magnetisierbaren und/oder magnetischen Partikel des ersten Teils 2 in Kontakt mit oder in der Nähe zu dem Heizmantel 14 positioniert. Die dort positionierten magnetisierbaren und/oder magnetischen Partikel werden durch den Heizmantel 14 auf einen Temperatur Tp3 oberhalb ihrer Curie-Temperatur Tc erwärmt, gehen damit in den zweiten Teil 3 der magnetisierbaren und/oder magnetischen Partikel über, stehen nicht mehr unter dem Einfluss des Magnetfeldes und werden von der gerührten fluiden Phase 4 wieder mitgerissen und selbstregulierend entsprechend ihrer Temperatur von dem Heizmantel 14 entfernt und dispergiert.

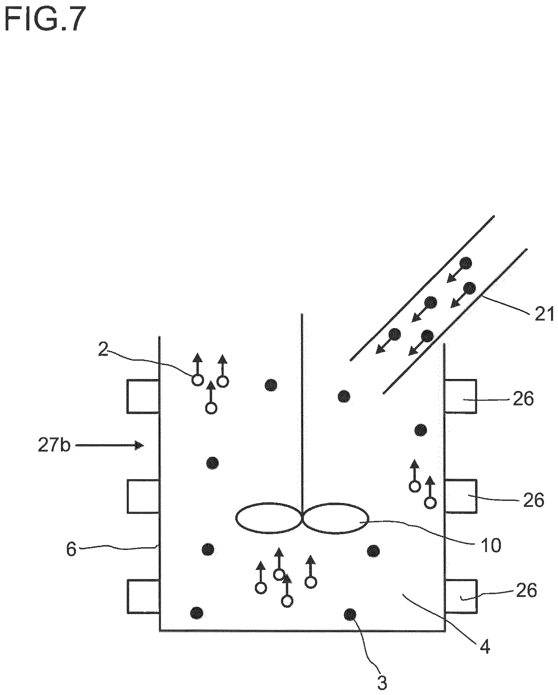

Figur 7 zeigt eine schematische Darstellung einer endothermen Reaktion in einem Batchreaktor mit selbstregulierender Temperaturführung und magnetischer Förderung.

In einem in Figur 7 dargestellten dritten Batchreaktor 27b wird wie anhand Figur 6 ausgeführt eine endotherme Reaktion durchgeführt. Jedoch befinden sich an der Reaktorwand 6 an Stelle der mindestens einen Magnetspule 5 und des Heizmantels 14 Magnetspulen 26 zur Erzeugung von Magnetfeldern einer magnetischen Förderung. Ein erster Teil 2 der magnetisierbaren und/oder magnetischen Partikel steht unter Einfluss der Magnetfelder der Magnetspulen 26 und wird mittels der magnetischen Förderung aus einem Reaktionsgemisch, bestehend mindestens aus der fluiden Phase 4 und den magnetisierbaren und/oder magnetischen Partikeln, entfernt. Außerhalb des dritten Batchreaktors 27b können die magnetisierbaren und/oder magnetischen Partikel des ersten Teils 2 wieder auf eine Temperatur Tp3 oberhalb ihrer Curie-Temperatur Tc erwärmt und beispielsweise über eine Zuführleitung 21 in den dritten Batchreaktor 27b zurückgeführt werden. Alternativ oder ergänzend können frische magnetisierbare und/oder magnetische Partikel mit einer Temperatur Tp3 oberhalb ihrer Curie-Temperatur Tc dem dritten Batchreaktor 27b über die Zuführleitung 12 zugeführt werden.

In Ergänzung zu den anhand der Figuren 1 bis 7 ausgeführten Ausführungsformen ergeben sich weitere Ausführungsformen dadurch, dass die magnetisierbaren und/oder magnetischen Partikel, also sowohl der erste Teil 2 als auch der zweite Teil 3, keine katalytische Aktivität aufweisen, sondern lediglich zum Wärmetransport eingesetzt werden oder dadurch, dass die magnetischen Eigenschaften der magnetisierbaren und/oder magnetischen Partikel durch eine Änderung ihrer Zusammensetzung wie eine Änderung der Oxidationsstufe anstelle einer Temperaturveränderung bewirkt wird.

Beispiel 1 Herstellung eines Feststoffs zur Verwendung in magnetischen Partikeln

Es wurden drei Proben, M1, M2 und M3, auf unterschiedliche Weise hergestellt. Alle Proben enthielten Eisenoxid, Kupferoxid und Nickeloxid. Alle Proben bestanden dabei aus 67,3 Gew.-% $Fe_2O_3$, 20,11 Gew.-% CuO und 12,59 Gew.-% NiO. Zur Herstellung von M1 wurden die Komponenten $Fe_2O_3$, CuO und NiO im genannten Verhältnis gemischt, im Mörser gemahlen und bei 900°C für 9 Stunden kalziniert. Zur Herstellung von M2 wurden $NiCO_3 \cdot 2Ni(OH)_2$, $CuCO_3 \cdot Cu(OH)_2$ und $Fe(NO_3)_3 \cdot 9H_2O$ gemahlen, erst bei 120°C für 16 Stunden und dann bei 150°C für 16 Stunden getrocknet und dann bei 250°C für 2 Stunden, dann bei 400°C für 2 Stunden und dann bei 550°C für 2 Stunden thermisch zersetzt um die entsprechenden Oxide zu halten. Anschließend wurde bei 900°C für 9 Stunden kalziniert. Zur Herstellung von M3 wurde eine Kofällung der entsprechenden Metallhydroxide mit KOH bei einem pH von 10 und einer Temperatur von 80°C durchgeführt. Anschließend wurde bei 100°C für 16 Stunden getrocknet und bei 350°C für 4 Stunden kalziniert. Um die Härte der Proben zu erhöhen wurden alle Materialien tablettiert und anschließend auf eine Partikelgröße zwischen 100 $\mu$m und 300 $\mu$m gemahlen.

Beispiel 2 Katalytische Aktivität der magnetischen Partikel

Die katalytische Aktivität der Proben M2 und M3 wurde untersucht. Dazu wurden jeweils 2,5 g einer Probe in eine Glasflasche geben und 90 g Nitrobenzol und 90 g Anilin zugegeben. Die Mischung wurde in einem Autoklaven mit Stickstoff gespült und dann mit Wasserstoff bei 35 bar und unter Rühren bei 200 min$^{-1}$ auf 130°C erwärmt. Sobald die Temperatur von 130°C erreicht wurde, wurde die Rührergeschwindigkeit auf 1300 min$^{-1}$ erhöht um die Reaktion zu starten.

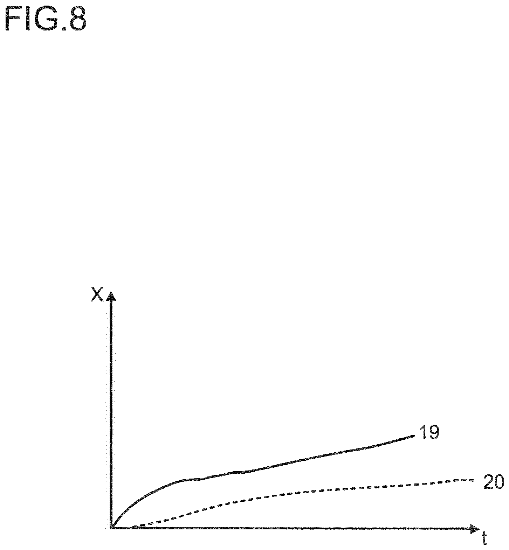

Die Ergebnisse sind in Figur 8 dargestellt. Hierzu wurde der Umsatz 17 von Nitrobenzol in Prozent als Funktion der Zeit t in Stunden aufgetragen. Sowohl der Umsatz, der mit Graph 19 für die Probe M3 dargestellt ist, als auch der Umsatz, der mit Graph 20 für die Probe M2 dargestellt ist, nahm mit fortschreitender Zeit zu, wobei für die Probe M3 in gleicher Zeit ein höherer Umsatz erzielt wurde. Die untersuchten Ni-Cu-Ferrite zeigten somit eine Aktivität bezüglich der Hydrierung von Nitrobenzol.

Beispiel 3 Änderung der magnetischen Eigenschaften magnetischer Partikel

Um die magnetischen Eigenschaften der Proben qualitativ zu beobachten wurde ein Pendel-Experiment durchgeführt. Ein Pendel aus nicht-magnetischem Material wurde mit magnetischem Proben-Material befüllt. Durch ein Magnetfeld, das von einem Dauermagneten erzeugt wurde, wurde das Pendel aus seiner Ruhelage gebracht. Das aus der Ruhelage gebrachte Pendel wurde mit heißer Luft mit einer Temperatur von 600°C, erzeugt von einem Gasbrenner, erwärmt. Die Temperatur der Probe wurde kontinuierlich mit einem Thermoelement gemessen, das gleichzeitig als Pendelaufhängung diente. Die Temperatur des Dauermagneten wurde dabei als konstant angenommen.

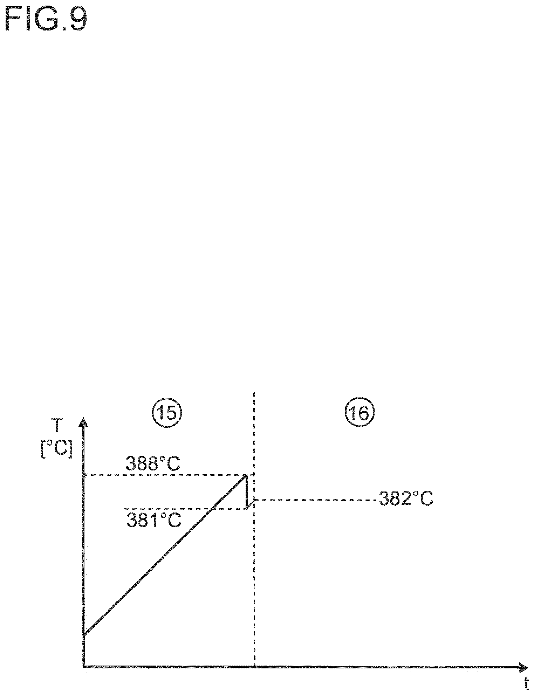

Sobald die Curie-Temperatur des Probenmaterials durch die Erwärmung mit heißer Luft überschritten war, verlor das Probenmaterial seine magnetischen Eigenschaften und das Pendel kehrte in seine Ruhelage zurück, da es nicht länger vom Dauermagneten beeinflusst wurde. Der Temperaturverlauf über die Zeit t in Minuten für die Probe M1 ist in Figur 9 dargestellt. Die Temperatur der Probe M1 stieg zunächst kontinuierlich bis auf 388°C an, wo sie sprunghaft auf 381°C absank und dann wieder auf 382°C anstieg. An dieser Stelle verlor der Dauermagnet seinen Einfluss auf die Probe und das Pendel den Kontakt zum Dauermagneten. Im dargestellten Bereich 15 steht die Probe unter Einfluss des Dauermagneten, was für den dargestellten Bereich 16 nicht der Fall ist. Die sprunghafte Temperaturveränderung ist auf den magnetokalorischen Effekt zurückzuführen, der auftritt sobald die Curie-Temperatur erreicht wird. Die Curie-Temperatur der Probe M1 wurde damit auf ungefähr 388°C bestimmt.

Die Ablösung des Pendels vom Dauermagneten gelingt auf diese Weise, wenn die Curie-Temperatur des Probenmaterials ungefähr zwischen 350°C und 400°C liegt. Die Probe M2 konnte nicht durch den beschriebenen Wärmeeintrag vom Dauermagneten gelöst werden und die Curie-Temperatur der Probe M2 wurde durch alternative Messmethoden auf 540°C bestimmt.

Die Probe M3 zeigte keine magnetischen Eigenschaften gegenüber dem Dauermagneten und wurde nicht vom Dauermagneten angezogen.

Zur quantitativen Bestimmung der Curie-Temperatur der Probe M2 wurde Thermogravimetrie unter Einsatz eines Dauermagneten, dynamische Differenzkalorimetrie und eine Hochfrequenz-Induktivitätsmessung eingesetzt.

In Abhängig von der Herstellungsweise wiesen die drei Proben M1, M2 und M3 unterschiedliche magnetische Eigenschaften auf. Die Curie-Temperatur der hergestellten Feststoffe konnte beeinflusst werden. M1 und M2 wurden darüber hinaus nochmals bei höherer Temperatur kalziniert, wodurch die Curie-Temperaturen beeinflusst werden konnten. Die durch die Kalzinierung erzielte Abnahme der Curie-Temperatur resultierte aus Umstrukturierungsprozessen in der Probe.

Vergleichsbeispiel 1 Herstellung von Anilin

Die Herstellung von Anilin aus Nitrobenzol wird wie beispielsweise in der WO 2010/130604 A2 beschrieben unter Verwendung eines Kupfer-Katalysators durchgeführt. Die exotherme Reaktion wird in einem Wirbelschichtreaktor mit internem Wärmetauscher durchgeführt. Die Wirbelschicht wird von einem Wasserstoff als Edukt enthaltenden Fluid durchströmt. Das Nitrobenzol wird flüssig in die Wirbelschicht eingedüst. Bei der Durchführung der Reaktion auf diese Weise ist der Wärmeübergang limitierend.

Beispiel 4 Herstellung von Anilin

Die Reaktion wird wie im Vergleichsbeispiel 1 durchgeführt, jedoch wird kein Wirbelschichtreaktor mit internem Wärmetauscher verwendet, sondern eine magnetisch unterstütze Wirbelschicht und der Katalysator ist in magnetischen Katalysatorpartikeln enthalten. Die Curie-Temperatur Tc der magnetischen Katalysatorpartikel beträgt 350 °C. Besitzen die magnetischen Partikel eine Temperatur, die niedriger als ihre Curie-Temperatur ist, so werden die magnetischen Partikel mit Hilfe eines angelegten Magnetfeldes in der Wirbelschicht lokalisiert. Werden die magnetischen Partikel in der Wirbelschicht auf eine Temperatur, die höher als ihre Curie-Temperatur ist, erwärmt, so werden die magnetischen Partikel mit der fluiden Phase aus der Wirbelschicht ausgetragen. Auf diese Weise wird mit den magnetischen Partikeln Wärme und Katalysatormasse aus der Wirbelschicht ausgetragen. Die Menge der ausgetragenen Wärme ist dabei selbst regulierend, da mehr Wärme ausgetragen wird, je mehr magnetische Partikel auf eine Temperatur erwärmt werden, die höher als ihre Curie-Temperatur Tc ist.

Bezugszeichenliste

| | |
|---|---|
| 1 | erste Wirbelschicht |
| 1a | zweite Wirbelschicht |
| 1b | dritte Wirbelschicht |
| 1c | vierte Wirbelschicht |
| 2 | erster Teil magnetisierbarer und/oder magnetischer Partikel mit einer Temperatur Tp2, die geringer ist als ihre Curie-Temperatur Tc |
| 3 | zweiter Teil magnetisierbare und/oder magnetische Partikel mit einer Temperatur Tp3, die höher ist als ihre Curie-Temperatur Tc |
| 4 | fluide Phase |
| 5 | Magnetspule |
| 6 | Reaktorwand |
| 7 | Strömungsrichtung der fluiden Phase 4 |
| 8 | Bewegungsrichtung des zweiten Teils 3 der magnetisierbaren und/oder magnetischen Partikel |
| 9 | Magnetspule zur Erzeugung eines alternierenden Magnetfeldes |
| 10 | Rührer |
| 11 | Bewegungsrichtung des ersten Teils 2 der magnetisierbaren und/oder magnetischen Partikel |
| 12 | Heizelement |
| 13 | Kühlmantel |
| 14 | Heizmantel |
| 15 | Bereich, in dem die Probe unter Einfluss des Dauermagneten steht |
| 16 | Bereich, in dem die Probe nicht unter Einfluss des Dauermagneten steht |
| 19 | Graph zur Probe M3 |
| 20 | Graph zur Probe M2 |
| 21 | Zuführleitung |
| 22 | Schwerkraft |
| 23 | Gitter |
| 24 | Fallrohr |
| 25 | Steigrohr |
| 26 | Magnetspule zur Erzeugung von Magnetfeldern einer magnetischen Förderung |
| 27 | erster Batchreaktor |
| 27a | zweiter Batchreaktor |
| 27b | dritter Batchreaktor |
| X | Umsatz von Nitrobenzol in [%] |
| t | Zeit in [h] |

**Patentansprüche**

1. Verfahren zur Selbstregulation eines Systems, umfassend folgende Schritte:

   (I) Nutzung eines Magnetfelds um magnetisierbare und/oder magnetische Partikel aus einem Bilanzraum zu transportieren oder in dem Bilanzraum zu lokalisieren, wobei die magnetisierbaren und/oder magnetischen Partikel

   a. im Bilanzraum aufgrund des Magnetfeldes lokalisiert werden, wenn diese ferromagnetische Eigenschaften aufweisen, und aus dem Bilanzraum mittels eines strömenden Fluids oder mittels Schwerkraft ausgetragen werden, wenn diese paramagnetische Eigenschaften aufweisen, oder
   b. unter Einfluss des Magnetfeldes aus dem Bilanzraum ausgetragen werden, wenn diese ferromagnetische Eigenschaften aufweisen, und in dem Bilanzraum lokalisiert werden, wenn diese paramagnetische Eigenschaften aufweisen,

   (II) Änderung von magnetischen Eigenschaften der magnetisierbaren und/oder magnetischen Partikel, die ferromagnetisch oder paramagnetisch sind, in dem Bilanzraum durch Änderung einer Temperatur $T_p$ der magnetisierbaren und/oder magnetischen Partikel oder durch Änderung der Zusammensetzung der magnetisierbaren und/oder magnetischen Partikel, wobei in dem Bilanzraum mindestens eine chemische Reaktion durchgeführt wird.

2. Verfahren nach Anspruch 1, wobei das Magnetfeld ein bewegtes Magnetfeld ist.

3. Verfahren nach Anspruch 1 oder 2, wobei die Temperatur $T_p$ zunimmt und die magnetisierbaren und/oder magnetischen Partikel aus dem Bilanzraum transportiert werden, wenn ihre Temperatur $T_p$ höher ist als ihre Curie-Temperatur $T_c$.

4. Verfahren nach einem der Ansprüche 1 bis 3, wobei die mindestens eine chemische Reaktion eine exotherme Reaktion ist.

5. Verfahren nach Anspruch 4, wobei die Temperatur $T_p$ zunimmt und mindestens ein Teil der Energie zur Erhöhung der Temperatur $T_p$ von der exothermen Reaktion freigesetzt wird.

6. Verfahren nach einem der Ansprüche 1 bis 3, wobei die Temperatur $T_p$ abnimmt und die magnetisierbaren und/oder magnetischen Partikel aus dem Bilanzraum transportiert werden, wenn ihre Temperatur $T_p$ niedriger ist als ihre Curie-Temperatur $T_c$.

7. Verfahren nach einem der Ansprüche 1 bis 3 oder 6, wobei die mindestens eine chemische Reaktion eine endotherme Reaktion ist.

8. Verfahren nach Anspruch 7, wobei die Temperatur $T_p$ abnimmt und mindestens ein Teil der Energie, welche von den magnetisierbaren und/oder magnetischen Partikeln abgegeben wird, zur Durchführung der endothermen Reaktion genutzt wird.

9. Verfahren nach einem der Ansprüche 1 bis 8, wobei die magnetisierbaren und/oder magnetischen Partikel die mindestens eine chemische Reaktion katalysieren oder ein katalytisch aktives Material enthalten.

10. Verfahren nach einem der Ansprüche 1 bis 8, wobei die magnetisierbaren und/oder magnetischen Partikel ein Edukt oder ein Produkt der mindestens einen chemischen Reaktion sind oder ein Edukt oder ein Produkt der mindestens einen chemischen Reaktion enthalten.

11. Verfahren nach einem der Ansprüche 1 bis 10, wobei die magnetisierbaren und/oder magnetischen Partikel nach dem Transport aus dem Bilanzraum gekühlt oder erwärmt und/oder regeneriert und in den Bilanzraum zurückgeführt werden.

12. Verfahren nach einem der Ansprüche 1 bis 2, 4, 7 oder 9 bis 11, wobei sich die Zusammensetzung der magnetisierbaren und/oder magnetischen Partikel dadurch ändert, dass durch die mindestens eine chemische Reaktion eine Oxidationsstufe mindestens einer Komponente der magnetisierbaren und/oder magnetischen Partikel verändert wird.

13. Verfahren nach einem der Ansprüche 1 bis 12, wobei der Bilanzraum Teil eines Festbettreaktors, eines expanded-bed-Reaktors, eines Wirbelschichtreaktors oder eines Suspensionsreaktors ist.

14. Verfahren nach einem der Ansprüche 1 bis 13, wobei die mindestens eine chemische Reaktion eine Hydrierung ist.

15. Verfahren nach einem der Ansprüche 1 bis 14, wobei eine Differenz zwischen der Curie-Temperatur Tc der magnetisierbaren und/oder magnetischen Partikel und einer Reaktionstemperatur Tr der mindestens einen chemischen Reaktion nicht mehr als 150 K beträgt.

16. Verfahren nach einem der Ansprüche 5, 6, 10, 12, oder 14 bis 15, wobei die exotherme Reaktion bei einer Regelreaktionstemperatur Trr durchgeführt wird, die niedriger ist als eine Grenztemperatur Tg, die Curie-Temperatur Tc der magnetisierbaren und/oder magnetischen Partikel so eingestellt ist, dass die Curie-Temperatur Tc der magnetisierbaren und/oder magnetischen Partikel größer ist als die Reaktionstemperatur Tr und kleiner ist als die Grenztemperatur Tg und eine Differenz zwischen der Curie-Temperatur Tc der magnetisierbaren und/oder magnetischen Partikel und der Grenztemperatur Tg nicht mehr als 20°C beträgt und sich die magnetischen Eigenschaften der magnetisierbaren und/oder magnetischen Partikel nur ändern, wenn die Reaktionstemperatur Tr, die zu Anfang gleich der Regelreaktionstemperatur Trr ist, zunimmt und dadurch die Temperatur Tp der magnetisierbaren und/oder magnetischen Partikel zunimmt und die Temperatur Tp der magnetisierbaren und/oder magnetischen Partikel die Curie-Temperatur Tc überschreitet.

## Claims

1. A method for self-regulation of a system comprising the steps of:

   (I) utilizing a magnetic field to transport magnetizable and/or magnetic particles out of a control volume or to localize said particles in the control volume, wherein the magnetizable and/or magnetic particles a. are localized in the control volume on account of the magnetic field when said particles have ferromagnetic properties and are discharged from the control volume by means of a flowing fluid or by means of gravity when said particles have paramagnetic properties or b. are discharged from the control volume under the influence of the magnetic field when said particles have ferromagnetic properties and are localized in the control volume when said particles have paramagnetic properties,
   (II) changing magnetic properties of the magnetizable and/or magnetic particles, which are ferromagnetic or paramagnetic, in the control volume by changing a temperature Tp of the magnetizable and/or magnetic particles or by changing the composition of the magnetizable and/or magnetic particles, wherein at least one chemical reaction is carried out in the control volume.

2. The method according to claim 1, wherein the magnetic field is a moving magnetic field.

3. The method according to claim 1 or 2, wherein the temperature Tp increases and the magnetizable and/or magnetic particles are transported out of the control volume when they have a temperature Tp higher than their Curie temperature Tc.

4. The method according to any one of claims 1 to 3, wherein the at least one chemical reaction is an exothermic reaction.

5. The method according to claim 4, wherein the temperature Tp increases and at least some of the energy for elevating the temperature Tp is liberated by the exothermic reaction.

6. The method according to any one of claims 1 to 3, wherein the temperature Tp decreases and the magnetizable and/or magnetic particles are transported out of the control volume when they have a temperature Tp lower than their Curie temperature Tc.

7. The method according to any one of claims 1 to 3 or 6, wherein the at least one chemical reaction is an endothermic reaction.

8. The method according to claim 7, wherein the temperature Tp decreases and at least some of the energy emitted by the magnetizable and/or magnetic particles is utilized for carrying out the endothermic reaction.

9. The method according to any one of claims 1 to 8, wherein the magnetizable and/or magnetic particles catalyze the at least one chemical reaction or comprise a catalytically active material.

10. The method according to any one of claims 1 to 8, wherein the magnetizable and/or magnetic particles are a reactant or a product of the at least one chemical reaction or comprise a reactant or a product of the at least one chemical reaction.

11. The method according to any one of claims 1 to 10, wherein once transported out of the control volume the magnetizable and/or magnetic particles are cooled or heated and/or regenerated and recycled into the control volume.

12. The method according to any one of claims 1 to 2, 4, 7 or 9 to 11, wherein the composition of the magnetizable and/or magnetic particles changes by the at least one chemical reaction changing an oxidation state of at least one component of the magnetizable and/or magnetic particles.

13. The method according to any one of claims 1 to 12, wherein the control volume is part of a fixed bed reactor, an expanded bed reactor, a fluidized bed reactor or a suspension reactor.

14. The method according to any one of claims 1 to 13, wherein the at least one chemical reaction is a hydrogenation.

15. The method according to any one of claims 1 to 14, wherein the difference between the Curie temperature Tc of the magnetizable and/or magnetic particles and a reaction temperature Tr of the at least one chemical reaction is no more than 150 K.

16. The method according to any one of claims 5, 6, 10, 12, or 14 to 15, wherein the exothermic reaction is carried out at a control reaction temperature Tcr which is lower than a threshold temperature Tt, the Curie temperature Tc of the magnetizable and/or magnetic particles has been adjusted such that the Curie temperature Tc of the magnetizable and/or magnetic particles is higher than the reaction temperature Tr and lower than the threshold temperature Tt and a difference between the Curie temperature Tc of the magnetizable and/or magnetic particles and the threshold temperature Tt is no more than 20°C and the magnetic properties of the magnetizable and/or magnetic particles only change when the reaction temperature Tr, which is initially equal to the control reaction temperature Tcr, increases causing the temperature Tp of the magnetizble and/or magnetic particles to increase and the temperature Tp of the magnetizable and/or magnetic particles to exceed the Curie temperature Tc.

## Revendications

1. Procédé d'autorégulation d'un système, comprenant les étapes suivantes :

(I) l'utilisation d'un champ magnétique pour transporter des particules magnétisables et/ou magnétiques hors d'une chambre de bilan ou les localiser dans la chambre de bilan, les particules magnétisables et/ou magnétiques

a. étant localisées dans la chambre de bilan en raison du champ magnétique lorsque celles-ci présentent des propriétés ferromagnétiques, et étant déchargées hors de la chambre de bilan au moyen d'un fluide en écoulement ou au moyen de la force de pesanteur lorsque celles-ci présentent des propriétés paramagnétiques, ou
b. étant déchargées de la chambre de bilan sous l'effet du champ magnétique lorsque celles-ci présentent des propriétés ferromagnétiques et étant localisées dans la chambre de bilan lorsque celles-ci présentent des propriétés paramagnétiques,

(II) la modification des propriétés magnétiques des particules magnétisables et/ou magnétiques qui sont ferromagnétiques ou paramagnétiques dans la chambre de bilan par modification d'une température Tp des particules magnétisables et/ou magnétiques ou par modification de la composition des particules magnétisables et/ou magnétiques, au moins une réaction chimique étant réalisée dans la chambre de bilan.

2. Procédé selon la revendication 1, dans lequel le champ magnétique est un champ magnétique déplacé.

**3.** Procédé selon la revendication 1 ou 2, dans lequel la température Tp augmente et les particules magnétisables et/ou magnétiques sont transportées hors de la chambre de bilan lorsque leur température Tp est supérieure à leur température de Curie Tc.

**4.** Procédé selon l'une quelconque des revendications 1 à 3, dans lequel ladite au moins une réaction chimique est une réaction exothermique.

**5.** Procédé selon la revendication 4, dans lequel la température Tp augmente et au moins une partie de l'énergie pour l'élévation de la température Tp est libérée par la réaction exothermique.

**6.** Procédé selon l'une quelconque des revendications 1 à 3, dans lequel la température Tp diminue et les particules magnétisables et/ou magnétiques sont transportées hors de la chambre de bilan lorsque leur température Tp est inférieure à leur température de Curie Tc.

**7.** Procédé selon l'une quelconque des revendications 1 à 3 ou 6, dans lequel ladite au moins une réaction chimique est une réaction endothermique.

**8.** Procédé selon la revendication 7, dans lequel la température Tp diminue et au moins une partie de l'énergie libérée par les particules magnétisables et/ou magnétiques est utilisée pour la réalisation de la réaction endothermique.

**9.** Procédé selon l'une quelconque des revendications 1 à 8, dans lequel les particules magnétisables et/ou magnétiques catalysent ladite au moins une réaction chimique ou contiennent un matériau catalytiquement actif.

**10.** Procédé selon l'une quelconque des revendications 1 à 8, dans lequel les particules magnétisables et/ou magnétiques sont un réactif ou un produit de ladite au moins une réaction chimique ou contiennent un réactif ou un produit de ladite au moins une réaction chimique.

**11.** Procédé selon l'une quelconque des revendications 1 à 10, dans lequel les particules magnétisables et/ou magnétiques sont refroidies ou réchauffées et/ou régénérées après le transport hors de la chambre de bilan, et recyclées dans la chambre de bilan.

**12.** Procédé selon l'une quelconque des revendications 1 à 2, 4, 7 ou 9 à 11, dans lequel la composition des particules magnétisables et/ou magnétiques se modifie en ce qu'un niveau d'oxydation d'au moins un composant des particules magnétisables et/ou magnétiques est modifié par ladite au moins une réaction chimique.

**13.** Procédé selon l'une quelconque des revendications 1 à 12, dans lequel la chambre de bilan fait partie d'un réacteur à lit fixe, 'un réacteur à lit expansé, d'un réacteur à lit fluidisé ou d'un réacteur à suspension.

**14.** Procédé selon l'une quelconque des revendications 1 à 13, dans lequel ladite au moins une réaction chimique est une hydrogénation.

**15.** Procédé selon l'une quelconque des revendications 1 à 14, dans lequel une différence entre la température de Curie Tc des particules magnétisables et/ou magnétiques et une température de réaction Tr de ladite au moins une réaction chimique n'est pas supérieure à 150 K.

**16.** Procédé selon l'une quelconque des revendications 5, 6, 10, 12 ou 14 à 15, dans lequel la réaction exothermique est réalisée à une température de réaction réglée Trr, qui est inférieure à une température limite Tg, la température de Curie Tc des particules magnétisables et/ou magnétiques est ajustée de sorte que la température de Curie Tc des particules magnétisables et/ou magnétiques soit supérieure à la température de réaction Tr et inférieure à la température limite Tg, et qu'une différence entre la température de Curie Tc des particules magnétisables et/ou magnétiques et la température limite Tg ne soit pas supérieure à 20 °C, et les propriétés magnétiques des particules magnétisables et/ou magnétiques ne se modifient que lorsque la température de réaction Tr, qui est au départ égale à la température de réaction réglée Trr, augmente et que la température Tp des particules magnétisables et/ou magnétiques augmente ainsi et que la température Tp des particules magnétisables et/ou magnétiques dépasse la température de Curie Tc.

FIG.1

# FIG.2

FIG.3

# FIG.4

FIG.5

# FIG.6

FIG.7

FIG.8

# FIG.9

**EP 3 160 633 B1**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 102007059967 A1
- DE 69603270 T2
- FR 2691718 A1
- WO 2006071527 A
- DE 3642557 A1
- US 4354856 A
- FR 2676374 A1
- EP 0115684 A1
- EP 0021854 A1
- US 4294688 A
- US 4292171 A
- DE 2950621 A1
- DE 102007059967
- WO 2008034770 A1
- WO 2010130604 A2

### In der Beschreibung aufgeführte Nicht-Patentliteratur

- **DONG et al.** Selective acetylene hydrogenation over core-shell magnetic Pd-supported catalysts in a magnetically stabilized bed. *American Institute of Chemical Engineers Journal,* 2008, vol. 54 (5), 1358-1364
- **FU et al.** Preparation and properties of magnetic alumina microspheres with a $\gamma$-Fe2O3/SiO2 core and Al2O3 shell. *Journal of Natural Gas Chemistry,* 2011, vol. 20 (1), 72-76

29